# EUROPEAN PATENT APPLICATION

(11) **EP 1 614 446 A2**
(43) Date of publication of application: **11.01.2006**
(21) Application number: 05077083.3
(22) Date of filing: 25.10.1996
(51) Int. Cl.: A61N 1/39, A61N 1/362

(54) **Electrically forcing cardiac output temporarily in tachycardia patients**

(30) Priority: 25.10.1995 US 548013; 25.10.1995 US 548014; 25.10.1995 US 548234
(62) Divisional of application: 96941319.4
(71) Applicant: Galvani Ltd., Eden Prairie, MN 55344 (US)
(72) Inventor: The designation of the inventor has not yet been filed
(74) Representative: Bryn-Jacobsen, Caelia

(57) **Abstract**

Upon detection of a cardiac arrhythmia (70), the apparatus delivers a series of pulses (72) followed by a further check for arrhythmia or other abnormal conditions. Upon the further detection of such abnormal conditions (74), the apparatus delivers a second series of pulses (78) and again checks for arrhythmia or other abnormal conditions (79). If such an abnormal condition is again sensed, a third series of pulses is applied (80). A further condition sensor senses the selection of continuous operation (82) and continues to apply to apply the third series of pulses to the patient.

## Description

The invention relates to an apparatus for temporarily electrically forcing cardiac output in the event of fibrillation resulting from an unsuccessful antitachycardia pacing therapy. The invention also relates to an apparatus for electrically forcing cardiac output by delivering a pulsed electric field to the heart. This field is sufficient to cause some cardiac output to maintain life temporarily but not necessarily sufficient to defibrillate the heart. Thus the device does not have the power (or size) of the common implantable defibrillators. Nevertheless, it could be made very small and thus serve as a temporary lifesaver for the high-risk patient to survive to an emergency room. These patients would then be candidates for the eventual implantation of an implantable defibrillator.

Many patients, especially those surviving myocardial infarction (heart attack), suffer from an occasional condition of ventricular tachycardia (VT). VT is a racing of the bottom chambers of the heart (the ventricles). It can be fatal although usually is not. This is in contrast to ventricular fibrillation (VF) which is nearly always fatal. While VT is usually not fatal it can cause fainting, loss of consciousness, anxiety on the part of the patient, and can occasionally degenerate into a lethal VF. Thus, while not a medical emergency of the level of VF, it is a condition that generally calls for prompt therapy.

In most cases, the VT can be abolished by application of low voltage pacing pulses of the appropriate timing. This is covered in many patents including U.S. # 4,408,606; U.S. # 4,398,536; U.S. # 4,488,553; U.S. # 4,488,554; and U.S. # 4,390,021 all assigned to Telectronics. Other patents dealing with antitachycardia pacing (ATP) include U.S. # 4,181,133 and U.S. # 4,280,502 assigned to Intermedics.

An antitachycardia pacemaker which was actually built and implanted in patients was the CyberTach 60 which was introduced in 1980 by Intermedics Inc. of Freeport, Texas. Its primary usage was for tachycardias of the atrium although it was implanted in a few patients for VT.

A later version of this device was the InterTach and InterTach 2 which added more sophisticated detection criteria and pacing output regimens. Another such device was the Tachylog of Pacesetter Inc. of Sylmar, California. Unfortunately, antitachycardia pacing is occasionally capable of accelerating the VT into a lethal VF. For the average patient this occurs on the order of a few percent of the time. If the patients are very carefully screened then this incidence can be reduced even further. However, due to the risk of killing the patients, these stand-alone antitachycardia pacemakers fell into disfavor. Some were implanted in patients along with a separate implantable defibrillator as backup in the event of acceleration into VF.

This was a very expensive solution and involved the implantation of bulky hardware into the patient. The present solution to this problem of VT is to implant the implantable cardioverter defibrillator (ICD) which includes antitachycardia pacing Since the ICD also has defibrillation included there is little concern over the occasional acceleration of the VT into a VF. Such a device is discussed in U.S. # 4,830,006 assigned to Intermedics.

The problem with such an approach is that the ICD remains a rather bulky device on the order of 100 cubic centimeters typical volume with the smallest being 60 cubic centimeters in volume. The reason for the large size is the large batteries and large capacitors which are required for the high energy defibrillation backup. Thus there is a need for a compact antitachycardia pacemaker with a different backup approach. Such a device could then hopefully be made much smaller and implanted in more patients.

Another condition often suffered by patients is atrial fibrillation. Although atrial fibrillation is not acutely life threatening it is a major cause of hospitalization. The lack of output from the atria can lead to the formation of clots in the atria. These clots, after they break loose, can then lodge in either the lungs (if they came from the right atrium) or in the brain - causing stroke - if they came from left atrium. Various drug approaches have been tried unsuccessfully for the treatment of atrial fibrillation. Some people are helped, but the majority of people are not safely treated by said drugs. One of the big side effects of the drugs is that they can lead to ventricular fibrillation which is nearly always fatal.

The atrial are much smaller than the ventricles and thus can be defibrillated with a much lower energy shock than then ventricles require. For that reason there has been a great deal of interest in atrial defibrillation. Some of the earliest patents for automatic defibrillators dealt with an atrial defibrillation system, for example US # 4,572,191. More recently the company InControl, Inc. has developed an implantable atrial defibrillator. For example US # 5,265,600 covers some aspects of an implantable atrial defibrillator.

The main concern over the use of the implantable atrial defibrillator is that the atrial defibrillation shock could lead to ventricular fibrillation. This is because a moderate level shock during the T-wave of the ventricle will typically lead to fibrillation. To avoid this problem, the InControl device senses the R-wave in the ventricle and carefully synchronises the shock so that it does not deliver a shock during the T-wave. See US # 5,350,402 assigned to InControl. The risk of fibrillating the heart with an atrial defibrillation shock can also be minimised by avoiding shocking after a certain combination of ventricular intervals as taught in US # 5,207,219 and US #5,282,837 of John Adams, both assigned to InControl.

Still, the risk remains that an atrial defibrillation shock will cause ventricular fibrillation and now the therapy for a non-lethal inconvenience has turned into a lethal condition. One possible solution is to include an atrial and ventricular defibrillation device in one package. Unfortunately, the energy required for ventricular fibrillation is significantly higher than is required for atrial defibrillation. Therefore, the capacitors and batteries must be so large that the device would end up being the same size as a conventional implantable cardiac defibrillator for the ventricle.

Thus, there is a definite need for a device which can perform atrial defibrillation yet which does not have the risk of killing the patient in the event that the atrial defibrillation shock accelerates the ventricles into a ventricular fibrillation rhythm.

As previously mentioned ventricular fibrillation is nearly always fatal. Approximately 400,000 Americans succumb to ventricular fibrillation each year. It is known that ventricular fibrillation, a usually fatal heart arrhythmia, can only be terminated by the application of an electrical shock delivered to the heart. This is typically done through electrodes applied to the chest by paramedics or other trained rescue squads using an external defibrillator which applies a shock of 200-360 joules. The voltage in such a shock is in the range of 1,000-5,000 volts. Such a shock can, if applied in time, abolish a fibrillation and restore the normal rhythm of the heart. Unfortunately, paramedics cannot usually respond rapidly enough with their external defibrillators to restore life. For example, in New York City, 99% of cardiac arrest patients die because the rescue squads cannot get the defibrillators to the victims quickly enough.

Once the patient has been saved, however, there is a very good treatment to increase the life expectancy. The device that delivers this therapy is the implantable cardioverter-defibrillator (ICD) which delivers a high energy shock of approximately 20-40 joules to the heart to abolish fibrillation and restore normal rhythm. Unfortunately, these devices are expensive (about $20,000) and are rather bulky with the smallest being 60 cubic centimetres.

Thus, it is not practical to implant an ICD in the heart of every high-risk patient. There is a need for a simple, inexpensive and extremely small device which could be implanted in high-risk cardiac patients to keep them alive until rescue squads can either defibrillate them or transport them to an emergency room. One aspect of the instant invention is essentially an electrical "paramedic in a can" which performs an electrical "CPR".

An object of the present invention is to overcome the problems with the prior art.

Various aspects of the invention are specified in the independent claims. Some preferred features are specified in the dependent claims.

One aspect of the present invention provides a device for stimulating cardiac cells, which are fibrillating, with pulses of appropriate duration, rate, and voltage so that a partial contraction of the heart is obtained. This then is used as a backup for antitachycardia pacing in the event that the ATP accelerates a VT into a VF. This electrical cardiac output forcing (ECOF) is sufficient to maintain life and consciousness on the part of the patient and thus allows ample time for the patient to be transported to an emergency room or for emergency rescue crews to arrive and perform an external defibrillation. At present, the only backup for any tachycardia pacing is the application of a large defibrillation shock. The generation of this large defibrillation shock requires the use of large capacitors and batteries.

According to another aspect of the invention there is provided a device for stimulating cardiac cells causing a partial contraction of the heart to force cardiac output during fibrillation or a hemodynamically compromising tachycardia. Electrical forcing fields are applied to the heart to give cardiac output on an emergency basis until the arrhythmia ceases or other intervention takes place. The device is a very small, low cost, implantable electrical unit with a lead set. The goal of this aspect of the invention is maintaining some cardiac output and not necessarily defibrillation. In the preferred embodiment a forcing field is generated by applying approximately 30-200 volts to the heart at a rate of approximately 100-180 pulses per minute. These fields are applied after detection of a hemodynamically significant arrhythmia and maintained for up to several hours. This will generate a cardiac output which is a fraction of the normal maximum capacity. The heart has a four or five times reserve capacity so a fraction of normal pumping activity will maintain life and consciousness. Various waveforms are used to optimise either electrical efficiency or patient comfort.

The device is implanted in high risk patients who have never had fibrillation. If the patients do later fibrillate, the Electrical Cardiac Output Forcing (ECOF) device forces a cardiac output for a period of up to several hours thus giving the patient enough time to get to a hospital. That patient would then be a candidate for an implantable cardioverter-defibrillator (ICD). The ECOF differs from the ICD in many respects. First, it is primarily intended for a single usage, forcing cardiac output over a period of hours, while the ICD is designed to furnish hundreds of defibrillation shocks over a period of years. Secondly, the ECOF is designed only to temporarily force cardiac output in spite of fibrillation while the ICD is designed to defibrillate so that the heart's normal rhythm can take over. Thirdly, the ECOF is designed to be implanted in high risk cardiac patients without any documented evidence of a hemodynamically compromising tachycardia or fibrillation while the ICD is generally reserved for those patients who have had a demonstrated severe tachycardia or fibrillation.

According to yet another aspect of the invention there is provided a device for applying electrical forcing fields to the ventricle to give cardiac output on an emergency basis until an arrhythmia ceases or other intervention takes place. The goal of this aspect of the invention is maintaining some ventricular output and not necessarily that of defibrillation. In a preferred embodiment a forcing field is generated by applying approximately 30-200 volts across the ventricles at a rate of approximately 100-180 beats per minute. These electrical fields are applied after detection of a ventricular fibrillation and maintained for up to several hours. This will generate a cardiac output which is a fraction of the normal maximum capacity. The heart has a four or five times reserve capacity so a fraction of normal pumping activity will maintain life and consciousness. Various waveforms can be used to optimise the electrical efficiency or patient comfort.

The device of this aspect of the invention has an electronic system and a lead system for delivering defibrillation shocks to the atrium. It also has a lead system and electronics for delivering electrical cardiac output forcing pulses to the ventricle in the event that the atrial defibrillation shock leads to ventricular fibrillation. If the atrial defibrillation shock does lead to ventricular fibrillation, the ECOF system will force cardiac output for a period of up to several hours thus giving the patient enough time to get to a hospital. This would also give rescue crews enough time to arrive and perform an external defibrillation.

One embodiment relates to stimulating cardiac cells, which are fibrillating, with pulses of appropriate duration, rate, and voltage so that a partial contraction of the heart obtains. This then is used as a backdrop for antitachycardia pacing in the event that the ATP accelerates a VT into a VF. This electrical cardiac output forcing (ECOF) is sufficient to maintain life and consciousness on the part of the patient and thus allows ample time for the patient to be transported to an emergency room or for emergency rescue crews to arrive and perform an external defribrillation. At present, the only backup for any tachycardia pacing is the application of a large defribrillation shock. The generation of this large defibrillation shock requires the use of large capacitors and batteries.

In one aspect there is an implantable device for treating atrial fibrillation with electrical therapy consisting of:
a. battery;
b. a high voltage generating circuit connected to the battery;
c. at least one small electrode for placement in a patient's heart connected to the pacing pulse generator circuitry;
d. control means connected to the pacing pulse generating circuit to generate pulses of appropriate timing to abolish the ventricular tachycardia according to the techniques of antitachycardia pacing;
e. a charging circuit connected to the battery capable of generating voltage pulses of 30-350 volts at a rate of at least 1 pulse per second;
f. a capacitor for storing energy from the charging circuit;
g. at least one large electrode for placement in a patient's heart; and
h. an output circuit for delivering pulses from the capacitor to at the least one large electrode.

so that, in the event that the antitachycardia pacing caused the ventricular tachycardia to accelerate into a lethal ventricular fibrillation, the large electrode pulses will electrically force cardiac output to maintain life until the patient could be externally defibrillated.

Preferably the large electrode has a greatest dimension of greater than one centimeter; and/or
the generated pulses of 30-350 V have at least one gradual edge with a rise time of greater than 100 microseconds.; and/or
each pulse has at least 6 narrow pulses inside it; and/or
the pacing pulse generating circuit battery is distinct from the charging circuit battery ; and/or
in which the pacing electrode is actually the same as the large electrode; and/or
in which the high voltage pulses are delivered at a rate of 60-200 pulses per minute.

The device includes:
a. providing a plurality of electrodes in or near the patient's heart;
b. detecting the presence of a tachycardia in the patient via said electrodes;
c. delivering electrical current pulses of a low voltage to the patient's heart via some of said electrodes after detecting the tachycardia;
d. monitoring for possible ventricular fibrillation;
e. in the event of the detection of ventricular fibrillation delivering higher voltage electrical pulses to the patient's heart via some of said electrodes at a rate between 60 and 200 pulses per minute, to directly force contraction in the patient's heart whereby a minimum level of cardiac output sufficient to maintain life is provided by said electrical current pulses.

Preferably each higher voltage pulse has gradual edges with rise times greater than 100 microseconds thereby minimizing patient discomfort and chest twitching.; and/or
further comprising the step of forming each higher voltage pulse of a train of at least 10 narrow pulses thereby minimizing patient discomfort and chest twitching; and/or
in which the higher voltage pulses have an amplitude of 30 - 350 volts.; and/or
in which the higher voltage pulses have a current of greater than 300 milliamperes; and/or
in which at least one of the electrodes has a dimension greater than 1cm ; and/or
in which the low voltage pulses of part c are delivered via the same electrodes as used for the higher voltage pulses of part e; and/or
comprising the additional step of automatically communicating with another party in the event of ventricular fibrillation.

According to one aspect there is a device, for implantation in the human body, for performing antitachycardia pacing, and for maintaining cardiac output of a patient's heart during a possible ventricular fibrillation induced by said antitachycardia pacing using electrical forcing fields comprising:
a. battery power supply means;
b. fibrillation detection means connected to said power supply means;
c. means to communicatively connect said battery power supply means and said arrhythmia detection means to the patient's heart; and
d. output control means connected to said arrhythmia detection means and to said battery power supply means and communicatively connected to the heart for delivering multiple electrical current pulses to the heart after detection of a fibrillation, said electrical current pulses having a voltage between 30 and 350 volts and current greater than 300 mA whereby contraction of the patient's heart is directly forced at a minimum level of cardiac output sufficient to maintain life as provided by said electrical current pulses. Preferably further comprising bradycardia output

pacing means; and/or
in which the output control means includes an inverter powered by said battery and driving a high energy capacitor; and/or
further comprising means to automatically alert another party in the event of ventricular fibrillation; and/or
further comprising means for storing programmable parameters for the detection of the arrhythmias, the antitachycardia pacing parameters, and the electrical cardiac output forcing parameters ; and/or
further comprising means to store the patient's internal electrical signals; and/or
further comprising blook pressure monitoring means connected to said arrhythmia detection means; and/or
in which said blood pressure monitoring means monitors cardiac output and further comprising the step of adjusting said electrical current pulse amplitude by said output control means to maintain a predetermined level of cardiac output based on blood pressure thereby conserving battery means.

In one embodiment there are
means to communicatively connect said battery supply means and said arrhythmia means to the patient's heart; and
output control means connected to said arrhythmia detection means and to said battery power supply means and to said means to communicatively connect to the human heart for delivering multiple electrical current pulses after the detection of tachyarrhythmia, said electrical current pulses having a voltage between 30 and 200 volts, whereby contraction in the patient's heart is directly forced and a minimum level of cardiac output sufficient to maintain life is provided by said electrical current pulses.

Preferably said electrical current pulses are delivered at a rate between 60 and 200 pulses per minute; and/or
the arrhythmia is a tachycardia.; and/or
the arrhythmia is fibrillation ; and/or
the arrhythmia is asystole ; and/or
further comprising blood pressure monitoring means connected to said arrhythmia detection means; and/or
further comprising means for monitoring the oxygen content of the blood ; and/or
in which said blood pressure monitoring means monitors cardiac output and further comprising the step of adjusting said electrical current pulse amplitude by said output control means to maintain a predetermined level of cardiac output thereby conserving electrical energy; and/or
in which said oxygen content measuring means monitors the O₂ content and further comprising the step of adjusting said electrical current pulse amplitude by said output control means to maintain predetermined level of cardiac output thereby conserving electrical energy ; and/or
wherein each electrical current pulse has rounded edges, thereby minimizing patient discomfort and chest twitching; and/or
further comprising the step of forming each electrical current pulse of a train of at least 10 narrow pulses, thereby minimizing patient discomfort and chest twitching ; and/or
in which said arrhythmia detection means reassesses the presence of arrhythmia at predetermined intervals and said electrical current pulses are stopped by said output control means if the arrhythmia is no longer present; and/or
further comprising the means to synchronize the ventricular electrical current pulses with the atrial depolarizations.; and/or
further comprising separate coil electrodes in the right atrium and the right ventricle whereby the atrial coil delivers an electrical pulse between 100 and 300 ms prior to the right ventricular pulse; and/or
further comprising means to independently vary the electrical current amplitudes of the separate atrial and ventricular electrodes; and/or
wherein said output control means delivers said electrical current pulses for at least one hour to maintain cardiac output.

In one aspect the device includes
(a) detecting the presence of the arrhythmia in a human heart; and
(b) delivering electrical current pulses to said human heart, wherein said electrical current pulses are delivered at a rate of between 60 and 200 pulses per minute, and wherein said predetermined electrical current pulses are strong enough to directly force contraction in parts of the patient's heart; thereby providing a level of cardiac output sufficient to maintain life in spite of the arrhythmia without necessarily defibrillating the patient.

Preferably said step of delivering electrical current pulses is repeated for at least one hour to maintain cardiac output.

According to another aspect there is a cardiac pacemaker comprising:
(a) a battery;
(b) arrhythmia detection circuitry connected to said battery;
(c) a voltage inverter connected to the battery to increase the voltage to a level of between 30 and 200 volts; and
(d) output circuitry, controlled by the arrhythmia detection circuitry connecting the voltage inverter to a patient's heart to deliver the inverter voltage repetitively in order to directly force a contraction in the patient's heart to maintain a minimum level of cardiac output in the event of an arrhythmia.

Preferably the repetitive inverter voltage is delivered at a rate between 60 and 200 pulses per minute; and/or
the output circuitry turns on gradually and thus provides at least one rounded edge; and/or
the output circuitry turns on gradually and thus provides a gently sloped leading edge; and/or
further comprising means to detect the atrial contraction and means to synchronize its output to the atrial contraction; and/or
in which the battery means has sufficient storage to provide the forcing pulses for at least one hour; and/or
in which the inverter output voltage is adjusted down to maintain cardiac output while minimising patient pain and battery drain; and/or
in which the appropriate timing is in synchronisation with the ventilator contraction; and/or
which at least one of the electrodes in the atria is used to conduct current in conjunction with at least one ventricular electrode for electrically forcing cardiac output.; and/or
in which the atrial current pulse width is shorter than and delivered prior to the ventricular current delivery.

According to one aspect, a device is arranged for
a. providing a plurality of electrodes in or near the patient's heart;
b. detecting the presence of atrial fibrillation in the patient via said electrodes;
c. delivering an electrical shock to the patient's heart via said electrodes after detecting the atrial fibrillation;
d. monitoring for possible ventricular fibrillation; and
e. in the event of the detection of ventricular fibrillation delivering current electrical pulses to the patient's heart via ventricular electrodes at a rate between 60 and 200 pulses per minute, said electrical current pulses having a strength between 30 and 375 volts to directly force contraction in the patient's ventricle whereby a minimum level of cardiac output sufficient to maintain life is provided by said electrical current pulses.

Preferably each electrical current pulse has gradual edges with rise times greater than 100 microseconds thereby minimizing patient discomfort and chest twitching; and/or
further comprising the step of forming each electrical current pulse of a train of at least 10 narrow pulses thereby minimizing patient discomfort and chest twitching; and/or
said electrical current pulses have a current in excess of 300 milliamperes and/or
the shock delivered after the detection of atrial fibrillation is synchronized to the ventricular contraction; and/or
the signal used to detect the presence of atrial fibrillation is stored in memory; and/or
in which the signal monitored for possible ventricular fibrillation is stored in memory; and/or
in which an atrial electrode is used to carry some of the current to directly force contraction in the patient's ventricle.

There is also a device for implantation in the human body, for performing atrial defibrillation and for maintaining cardiac output of a patient's heart during a possible ventricular fibrillation induced by said atrial defibrillation using electrical forcing fields comprising:
a. battery power supply means;
b. fibrillation detection means connected to said battery power supply means;
c. means to communicatively connect said battery power supply means and said fibrillation detection means to the patient's heart; and
d. output control means connected to said fibrillation detection means and to said battery power supply means and to said means to communicatively connect to the heart for delivering multiple electrical current pulses to the heart after detection of a ventricular fibrillation, said electrical current pulses having a voltage between 30 and 375 volts whereby contraction of the patient's heart is directly forced at a minimum level of cardiac output sufficient to maintain life as provided by said electrical current pulses.

Preferably the device further comprising bradycardia output pacing means; and/or
in which the output control means includes an inverter powered by said battery and driving a high energy capacitor; and/or
in which said output control means comprise a voltage multiplier powered by said battery; and/or
further comprising means for storing programmable parameters; and/or
further comprising means to store the patient's internal electrical signals; and/or
further comprising means to communicate with a device external to the body; and/or
further comprising means to report the battery status.

Preferably said large electrode includes dimensional parts which vary from a smaller section to a bigger section, the bigger section being greater than one centimeter; and/or
said generated voltage pulses of 30 to 375 V include at least one gradual rise profile such that said voltage pulses rising from 30 to 375 volts require at least 100 microseconds; and/or
each of said generated voltage pulses further includes at least six narrow pulses in parallel formation; and/or
said at least one electrode is same as said large electrode; and/or
wherein said at least one electrode adapted to be placed in the atrium is structured to cooperate with said large electrode to provide a current path to thereby trigger the electrical cardiac output; and/or
wherein the electrical cardiac output width is shorter than said voltage pulse applied to said at least one electrode adapted for placement in the atrium to direct said voltage pulse into the atrium and further said electrical cardiac output being sequenced to be deliverable prior to the voltage pulse being directed into the atrium.
A device is provided for a method of electrically controlling an atrial fibrillation in a patient wherein an atrial defibrillation therapy system results in ventricular fibrillation and an implantable device in cooperation with the atrial defibrillation therapy system supplies an electrical cardiac output upon sensing the onset of the ventricular fibrillation, comprising the device implemented steps of:
providing a plurality of electrodes adaptable to be placed at one of proximate to and inside a patient's heart;
detecting presence of atrial fibrillation in said patient's heart;
delivering an electrical shock to said patient's heart upon detection of an onset of said atrial fibrillation;
monitoring for possible ventricular fibrillation; and/or
delivering a series of electrical pulses to said patient's heart at a rate between 60 and 200 pulses per minute and at a voltage of between 30 and 375 volts to induce contraction in the patient's ventricle and to enable a minimum cardiac electrical output sufficient to maintain life.

Preferably each of said series electrical pulses includes a formation structure having gradual rise durations greater than 100 microseconds such that said pulses are adaptable for use in human patients wherein said gradual rise minimizes patient discomfort and chest twitching; and/or
further comprising the step of forming each of said series of electrical pulses a segment of a discreet train of at least 10 narrow pulses to minimize patient discomfort and chest twitching; and/or
further comprising the step of delivering a current above 300 milliamperes to induce said electrical cardiac output; and/or
further comprising the step of storing atrial fibrillation sensing events in a microprocessor memory; and/or
further comprising the step of storing ventricular fibrillation sensing events in a microprocessor memory; and/or
in which one of said plurality of electrodes is adapted to be placed in the atrium of the patient's heart and further that said electrode is adapted to deliver said electrical pulses to the heart to induce contraction in the patient's heart.

There is also an implantable device including an atrial defibrillation system and a ventricular fibrillation control system wherein resultant ventricular fibrillation is caused by the atrial defibrillation system during a treatment of a heart patient, the implantable device comprising:
a power supply system;
means for detecting fibrillation having electrical connection to said power supply system;
means for integrating said power supply system and said means for detecting fibrillation to be adaptable for placement in the patient's heart; and
means for controlling electrical output connected to said means for detecting, said power supply system and said means for integrating to thereby deliver a series of electrical pulses to the heart upon detection of a ventricular fibrillation wherein said series of electrical pulses include a voltage between 30 and 375 volts to force a minimum level of cardiac output sufficient to maintain life.

Preferably further comprising means for pacing the heart for bradycardia wherein a slow heart beat is paced back to within a normal beat per minute range; and/or
in which said means for controlling electrical output includes a high energy capacitor and further includes an inverter powered by said power supply system; and/or
wherein said means for controlling includes a voltage multiplier powered by said power supply system; and/or
further comprising means for storing data including programmable parameters and the patient's detected internal electrical signals wherein said means for storing is implemented in a microprocessor to store and process the data; and/or
further comprising means for communicating with a device external to the patient's body such that a communication and data exchange structure is implemented between said implantable device and said external device; and/or
further comprising means for indicating operational status of said power supply system.

There is also provided an implantable device for maintaining cardiac output of a patient's heart during an onset of tachyarrhythmia wherein electrical forcing fields are applied to the heart until the onset of the tachyarrhythmia is eliminated, the device comprising:
a power supply system including a rechargeable battery;
means for detecting arrhythmia connected to said power supply system;
interface means for adapting to connect said supply system means and said means for detecting arrhythmia to the patient's heart; and
means for controlling electrical output connected to said means for detecting arrhythmia, said power supply system and said interface means to thereby deliver multiple electrical current pulses to the heart after detection of said tachyarrhythmia, wherein said electrical current pulses include a voltage between 30 and 200 volts, whereby contraction in the patient's heart is directly forced and a minimum level of cardiac output sufficient to maintain life is provided by the electrical current pulses

Preferably in which said electrical current pulses are delivered at a rate between 60 and 200 pulses per minute; and/or
which the arrhythmia is of an asystole type relating to absence of cardiac contraction and cardiac arrest; and/or
further comprising means for monitoring blood pressure wherein said monitoring means includes operable connections and communication with said arrhythmia detection means; and/or
further comprising means for monitoring blood oxygen content incorporated therein to provide the level of oxygen as part of an overall assessment of the patient's condition; and/or
wherein said blood pressure monitoring means monitors cardiac output and further includes means for adjusting amplitudes of said electrical current pulses in cooperation with said means for controlling output to maintain a predetermined level of cardiac output, thereby conserving electrical energy; and/or
wherein said means for monitoring blood oxygen content further includes means for adjusting amplitudes of said electrical current pulses in cooperation with said means for controlling output to maintain predetermined level of cardiac output, thereby conserving electrical energy; and/or
wherein each of said electrical current pulses includes rounded rise profiles with no sharp transitional rises, thereby minimizing patient discomfort and chest twitching; and/or
wherein each of said electrical current pulses includes a train of at least 10 narrow pulses, structured to maintain life without defibrillating the patient; and/or
delivering electrical current pulses is repeated for a duration of at least one hour to maintain cardiac output.

There is provided a cardiac pacemaker comprising:
a battery;
arrhythmia detection circuitry connected to said battery;
a voltage inverter connected to the battery to increase the voltage to a level of between 30 and 200 volts; and
output circuitry, controlled by the arrhythmia detection circuitry connecting the voltage inverter to a patient's heart to deliver the inverter voltage repetitively in order to directly force a contraction in the patient's heart to maintain a minimum level of cardiac output in the event of an arrhythmia.

Preferably the repetitive inverter voltage is delivered at a rate between 60 and 200 pulses per minute; and/or
the output circuitry turns on gradually and thus provides at least one rounded edge; and/or
the output circuitry turns on gradually and thus provides a gently sloped leading edge profile; and/or
further comprising means to detect the atrial contraction and means to synchronize its output to the atrial contraction; and/or
the battery means includes sufficient storage capacity to provide the forcing pulses for at least one hour; and/or
the inverter output voltage is adjusted down to maintain cardiac output while minimizing patient pain and battery drain.

There is provided an implantable device for treating ventricular tachycardia with electrical pacing therapy comprising:
a battery;
pacing pulse generating circuit connected to said battery;
at least one small electrode adapted for placement in a patient's heart connected to the pacing pulse generating circuit;
control means connected to the pacing pulse generating circuit to generate pulses of appropriate timing to abolish the ventricular tachycardia according to the techniques of antitachycardia pacing;
a charging circuit connected to the battery capable of generating voltage pulses of 30-350 volts at a rate of at least 1 pulse per second;
a capacitor for storing energy from the charging circuit;
at least one large electrode for placement in a patient's heart; and
an output circuit for delivering pulses from the capacitor to at the least one large electrode such that in the event the antitachycardia pacing causes the ventricular tachycardia to accelerate into a lethal ventricular fibrillation, the large electrode pulses will electrically force cardiac output to maintain life until the patient could be externally defibrillated.

Preferably the large electrode has a greatest dimension greater than one centimeter; and/or
the generated pulses of 30-350 V have at least one gradual rise profile with a time to rise relationship of greater than 100 microseconds per rise; and/or
in which each pulse comprises at least 6 narrow sub-pulses forming subsections therein; and/or
in which the pacing pulse generating circuit battery is distinct and separate from the charging circuit battery; and/or
in which the large electrode is also the pacing electrode; and/or
in which the high voltage pulses are delivered at a rate of 60-200 pulses per minute.

There is a device arranged for:
providing a plurality of electrodes adapted to be placed in one of proximate to and inside the patient's heart;
detecting the presence of a tachycardia in the patient via said electrodes;
delivering electrical current pulses of a low voltage to the patient's heart via at least one of said electrodes after detecting the tachycardia;
monitoring for possible ventricular fibrillation; and
delivering higher voltage electrical pulses to the patient's heart in the event of the detection of ventricular fibrillation via at least one of said electrodes at a rate between 60 and 200 pulses per minute, to directly force contraction in the patient's heart whereby a minimum level of cardiac output is provided by said electrical current pulses sufficient to maintain life.

Preferably each higher voltage pulse is set to maintain gradual rise profiles with rise durations greater than 100 microseconds thereby minimizing patient discomfort and chest twitching; and/or
further comprising the step of structuring each higher voltage pulse to form a train of at least 10 narrow pulses thereby minimizing patient discomfort and chest twitching; and/or
in which the higher voltage pulses have an amplitude of 30 - 350 volts; and/or
in which the higher voltage pulses have a current of greater than 300 milliamperes; and/or
in which at least one of the electrodes includes a physical dimension greater than 1 cm; and/or
in which the low voltage pulses are delivered via the same electrodes used for delivering the higher voltage pulses; and/or
comprising the additional step of automatically communicating with another party in the event of ventricular fibrillation.

There is also a device for implantation in a human body, for performing antitachycardia pacing, and for maintaining cardiac output of a patient's heart during a possible ventricular fibrillation induced by said antitachycardia pacing, the device comprising:
a battery power supply means;
fibrillation detection means connected to said power supply means;
means to communicatively connect said battery power supply means and said arrhythmia detection means to be adaptable to the patient's heart; and
output control means connected to said arrhythmia detection means and to said battery power supply means and communicatively adapted to the patient's heart for delivering multiple electrical current pulses to the heart after detection of a fibrillation, said electrical current pulses having a voltage between 30 and 350 volts and current greater than 300 mA whereby contraction of the patient's heart is directly forced at a minimum level of cardiac output sufficient to maintain life as provided by said electrical current pulses.

Preferably further comprising bradycardia output pacing means; and/or
in which the output control means includes an inverter powered by said battery and is structured to drive a high energy capacitor; and/or
further comprising means to automatically alert another party in the event of ventricular fibrillation; and/or
further comprising means for storing programmable parameters for the detection of the arrhythmias, the antitachycardia pacing parameters, and the electrical cardia output forcing parameters; and/or
further comprising means to store data relating to the patient's internal electrical signals; and/or
further comprising blood pressure monitoring means connected to said arrhythmia detection means; and/or
in which said blood pressure monitoring means monitors cardiac output and further comprises means for adjusting said electrical current pulse amplitude in cooperation with said output control means to maintain a predetermined level of cardiac output based on blood pressure levels to thereby conserve power.
**FIG. 1** is a block diagram illustrating a system constructed in accordance with the principles of the present invention.
**FIG. 2** shows a connection of an implantable embodiment of the device to the heart in an epicardial patch configuration.
**FIG. 3** shows the connection of an implantable embodiment of the device to the heart using an endocardial lead system and the device housing as an electrode.
**FIG. 4** is a diagram showing a representative pulsatile electrical signal.
**FIG. 5** is a flow chart illustrating one embodiment of the method of the invention.
**FIG. 6** is a diagram showing the expected effect of a 50 V pulse on the heart during diastole.
**FIG. 7** is a diagram showing the expected effect of a 50 V pulse on the heart during systole.
**FIG. 8** is a diagram showing the expected effect of a 50 V pulse on the heart during fibrillation
**FIG. 9** shows a high comfort rounded pulse that is useful for an electrical cardiac output forcing method and apparatus.
**FIG. 10** shows a pulse comprised of many smaller pulses that is also useful for an electrical cardiac output forcing method and apparatus.
**FIG. 11** is a block diagram illustrating a system constructed in accordance with the principles of the present invention.
**FIG. 12** shows an embodiment in which different batteries are used for the backup circuitry and the antitachycardia pacing circuitry.
**FIG. 13** shows an embodiment in which all pacing is done through the large electrodes.
**FIG. 14** shows a preferred embodiment in which a single small ventricular electrode is used.
**FIG. 15** shows the steps taken when using the device in which the invention is embodied.
**FIG. 16** depicts some possible cardiac output forcing pulses.
**FIG. 17** shows a sample antitachycardia pacing burst.
**FIG. 18** shows a complete system.
**FIG. 19** shows a basic atrial implantable defibrillator in the human body.
**FIG. 20** shows the basic lead system for such an atrial implantable defibrillator.
**FIG. 21** shows the basic schematic for an implantable atrial defibrillator.
**FIG. 22** shows the biphasic waveform that is used for atrial defibrillation.
**FIG. 23** shows the basic lead system for the device of this invention.
**FIG. 24** shows the basic waveforms of the electrically cardiac output forcing approach used for the backup in case of ventricular fibrillation.
**FIG. 25** shows the basic schematic of an apparatus in which one aspect of the invention is embodied.
**FIG. 26** shows a flow diagram of the steps taken when using the apparatus of Figure 25.
**FIG. 27** shows another apparatus in which the present invention is embodied that includes separate atrial and ventricular coil electrodes.
**FIG. 28** is a schematic of a basic ECOF system.
**FIG. 29** is a schematic of a more complex version of the ECOF device.
**FIG. 30** shows the ECOF implanted in the human body along with its lead system.
**FIG. 31** shows a flow chart of the method of use of the ECOF device.
**FIG. 32** shows a sample of the output waveforms

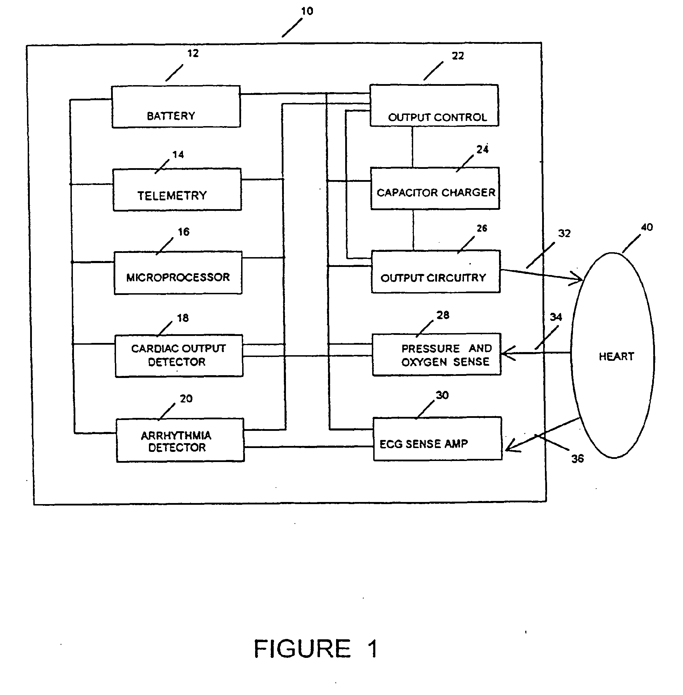

FIG 1 is a block diagram illustrating a system 10. The device circuitry is connected to the heart 40 via a series of leads: output lead 32, pressure sense lead 34, and ECG sense lead 36. The electronic circuit includes a conventional ECG amplifier 30 for amplifying cardiac signals. The amplified cardiac signals are analysed by a conventional arrhythmia detector 20 which determines if an arrhythmia is present. The arrhythmia detector 20 may be one of several types well known to those skilled in the art and is preferably able to distinguish between different types of arrhythmias. For example, fibrillation, tachycardia or asystole. The circuit also contains an optional pressure sensing and/or oxygen content sensing section 28 which amplifies and conditions a signal from an optional pressure or O₂ sensor from within the heart or artery. The output of the pressure/O₂ content sense circuit 28 is fed to a cardiac output detection circuit 18 which analyses the data and determines an estimate of the cardiac output. Data from the arrhythmia detector circuit 20 and the cardiac output detection circuit 18 is fed to the microprocessor 16. The microprocessor 16 determines if Electrical Cardiac Output Forcing (ECOF) is appropriate. If forcing is indicated, the microprocessor 16 prompts the output control 22 to charge a capacitor within the output circuit 26 via the capacitor charger 24. The output control 22 directs the output circuitry 26 to deliver the pulses to the heart 40 via the output leads 32. The microprocessor 16 may communicate with external sources via a telemetry circuit 14 within the device 10. The power for the device 10 is supplied by an internal battery 12.

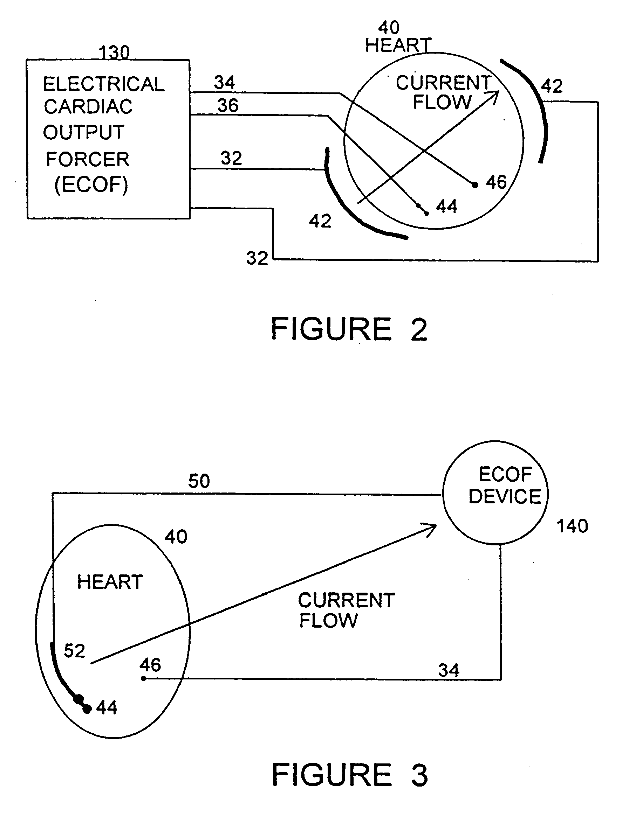

FIG. 2 is a diagram showing the connection of an implantable back up portion the device 130 to the heart 40 in an epicardial patch configuration. In this thoractomy configuration, current passes through an output lead pair 32 to electrode patches 42 which direct the current through the heart 40. There is an optional pressure sense lead 34 which passes the signal from an optional pressure transducer 46 which lies in the heart 40. The ECG is monitored by sense electrodes 44 and passed to the device 130 by a lead 36. The area of pressure transducer 46 which lies in the heart 40. The ECG is monitored by sense electrodes 44 and passed to the device 130 by a lead 36. The area of the electrodes 42 is at least 0.5cm². The size of the electrode is greater than that of a pacing lead and no more than that of a defibrillation electrode or between approximately 0.5cm² and 20cm² each.

**FIG. 3** shows a non-thoractomy system back up portion of the device 130. In this system, the current passes from a coil electrode 52 in the heart 40 to the housing of the device 140. An endocardial lead 50 combines the ECG sensing lead and the pulse output lead. The ECG is monitored by sense electrodes 44 in the heart 40 and passes through the endocardial 50. There is an optional pressure transducer or oxygen content sensor 46 in the heart 40 which passes a signal to the device 140 via optional lead 34.

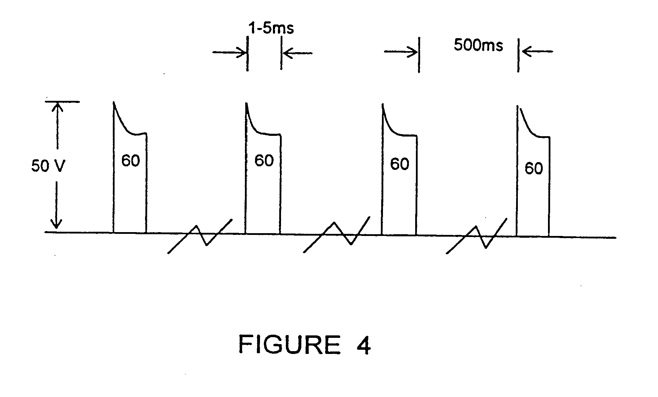

A series of forcing pulses 60 are shown in **FIG. 4**. The pulses are approximately 50 V in amplitude with a spacing of approximately 500 ms. The 50 V and the 500 ms pulse spacing are chosen as illustrative for an implantable embodiment. The forcing pulse interval is chosen to maximize cardiac output within the limits of the device circuitry and the response of the heart muscle. An interval of 500 ms corresponds to a heart rate of 120 beats per minute. This will produce a greater output than a typical resting rate of 60 beats per minute. However, a rate of 240 beats per minute would produce a lower output due to mechanical limitations of the heart. Thus a practical range of just 60 to 200 beats per minute is appropriate. The pulses could also be timed to coincide with the natural pumping of the atria, thus improving overall cardiac output.

The higher the voltage, the higher the forcing fields, and therefore a greater number of heart cells contracting producing greater cardiac output. However, the higher voltage produces greater patient discomfort and extraneous muscle twitching.

Implantable batteries are also limited to a certain power output and energy storage. If an output pulse is 50 V and the electrode impedance is 50 Ω, the power during the pulse is P = V²/R = 50 V* 50 V/50 Ω = 50 W. If the pulse has a duration of 2 ms then the energy per pulse is 0.1 J. If two pulses are delivered every second, the charger must be capable of delivering 0.2 J per second which is 200 mW. This is well within the limits of an implantable battery An implantable battery can typically deliver 5 W of power However, 200 V pulses at 3 per second would require 4.8 W which is near the limit of the battery and charging circuitry. A typical implantable battery energy capacity is 10,000 J. Delivering forcing pulses at a rate of 4.8 W would deplete the battery in only 35 minutes (10,000 J/4.8 W = 2083 seconds). Thirty five minutes may not be enough time to transport the patient to a hospital. Therefore 200 V represents the highest practical voltage for continuous operation in an implantable embodiment, although voltages of up to 350 V (maximum voltage for electrolytic capacitor) could be used for short periods and adjusted down when hemodynamic output is verified. A practical lower limit is about 10 V. During normal sinus rhythm, 10 V delivered through the patches would pace. However, during fibrillation the 10 V could not pace and only cells very near the electrodes would be captured. This would be insufficient for forcing cardiac output. A typical range would be 30 - 200 V, with an optional 350 V initial burst.

Assuming a compromise voltage of 30 V and an electrode impedance of 100 ohms, the forcing current would be 300 milliamperes although the system would provide more if necessary.

These calculations also suggest other differences between an implantable ECOF and an ICD. With a battery storing 10,000 J and an ECOF pulse having 0.1 J, this ECOF would be capable of delivering 100,000 pulses. An ICD can only deliver 200 - 400 shocks of about 30 J. The ECOF is also very different from an implantable pacemaker which typically delivers 150,000,000 pacing pulses (5 years at 60 BPM) each of about 0.00005 J.

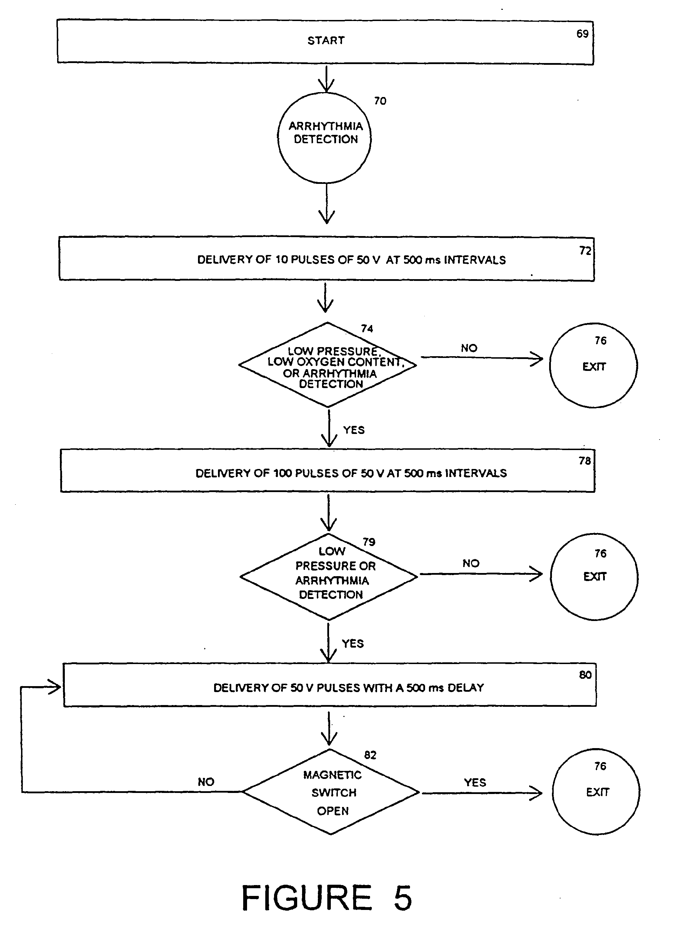

**FIG. 5** is a flowchart illustrating the method of the invention, which is provided for purposes of illustration only. One skilled in the art will recognize from the discussion that alternative embodiments may be employed without departing from the principles of the invention. The flow diagram shown in **FIG. 5** represents a method of automatically treating a heart which is in fibrillation, tachycardia, or asystole and thereby pumping inefficiently or not at all. Electrodes are attached 69. A diagnosis of the presence of an arrhythmia is made 70. A series of cardiac output forcing electric pulses 72 is automatically delivered. It should be understood that the therapy 72 may be delivered for any output compromising cardiac arrhythmia. After delivery of 10 forcing pulses (at a rate of 60 - 200 BPM) in the first block 72, the status of the heart is determined 74. If an arrhythmia is still present and there exists low pressure or low O₂ within the heart, more forcing pulses are delivered 78. In step 78 the amplitude of the electrical variable based on the optional blood pressure or oxygen content monitoring means. If the heart is pumping at a safe level, the therapy ceases and exits 76. Note that this means that the ECOF successfully defibrillated the patient's heart even though this is not a primary goal of the system. This could be tested in patients who were scheduled to receive an ICD, in a hospital setting. Those patients who are defibrillated by ECOF pulse therapy could then receive the ECOF instead of the larger ICD. After the therapy 78 has been delivered, the pressure and/or the 0₂ content and the ECG are again monitored 79. If the therapy 78 is successful, it ceases and exits 76. If the therapy 78 is unsuccessful in producing a safe level of pumping efficiency, the method proceeds to a continuous cardiac assist mode 80. The therapy may only be stopped by an external command, for example, a telemetry signal or a magnet which is applied to the chest activating a magnetic reed switch 82 which terminates the therapy and exits 76. To minimize patient discomfort and maximize battery life, the forcing voltage could be adjusted down when sufficient pressure signals or adequate flow measured by other means were detected, for example, the pressure sense transducer could be replaced by an oxygen detector or a doppler flow measuring device. The pulse rate could also be adjusted to maximize output.

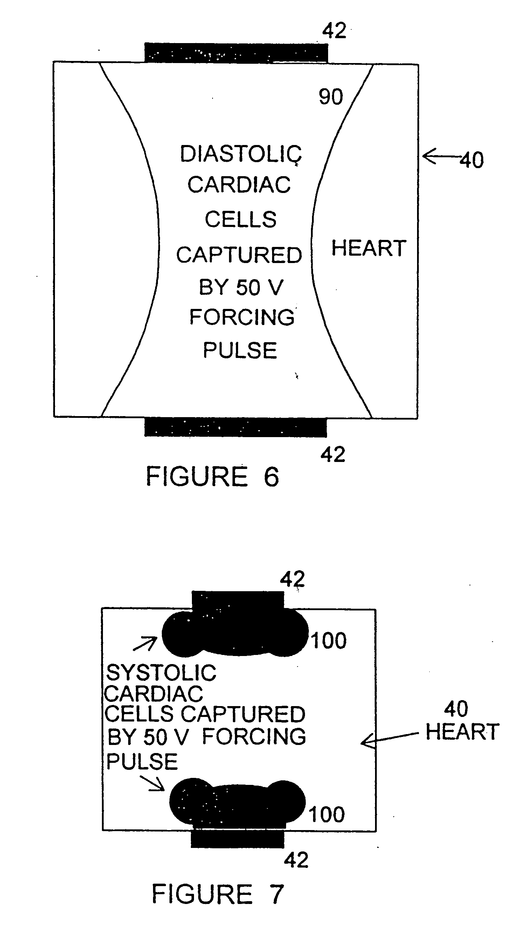

**FIG. 6** is a diagram showing the effect of a 50 V forcing pulse on the heart 40 during electrical diastole (cells at rest). The current is passed through the heart 40 by the electrodes 42. Approximately 60% of cardiac cells 90 would be captured by a 50 V pulse if the cells were in diastole. The captured cells 90 mostly lie in the direct path between the electrodes 42 and near the electrodes 42 where the field strengths are highest. Of course, over a time period of about 100 ms these directly captured cells then propagate an activation wavefront to stimulate the rest of the heart. This so called far-field pacing is not wholly relevant here as the hearts, of interest, are in fibrillation and not in diastole.

**FIG. 7** is a diagram showing the effect of a 50 V forcing pulse on the heart during electrical systole (cells already stimulated). The current is passed through the heart 40 by the electrodes 42. Approximately 20% of cardiac cells 100 would be captured by a 50 V pulse if the cells were in systole. The captured cells 100 are nearest each electrode 42 where the field strengths are highest. Capture in systolic cells means that their activation potential is extended. This capture requires significantly higher fields (typically 5 V/cm or 10 V/cm) than those required for diastolic cell capture (typically 0.5 V/cm or 1 V/cm).

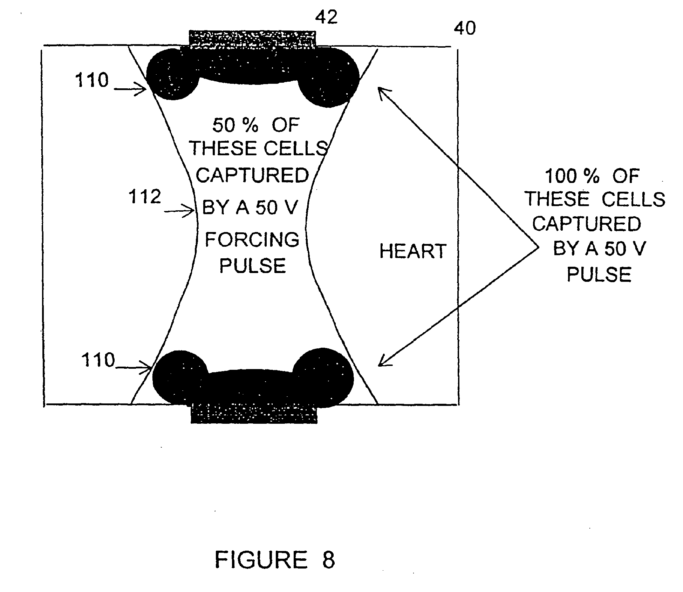

**FIG. 8** is a diagram showing the effect of a 50 V forcing pulse on the heart during fibrillation. During fibrillation there are always cells in systole and diastole simultaneously. But, the vast majority are in systole. The diagram assumes 50% of the cells are in diastole which applies only after several capturing pulses. The current is passed through the heart 40 by the electrodes 42. 100% of the cells 110 nearest the electrodes 42 would be captured due to the high field strength. As shown in **FIG. 7,** even systolic cells are captured by high field strengths. 50% of the cells 112 in the direct path between the electrodes 42 would be captured if it is assumed that 50% of all cells are in diastole. If roughly 60% of cardiac cells are captured by a 50 V pulse when the cells are in diastole, and 20% are captured when in systole, and if 50% are in systole and 50% in diastole, 40% would be captured during fibrillation. This calculation is shown in the following table. The last two columns give the resulting mechanical action and the contribution to cardiac output forcing.

Considering the cardiac cells that are originally in diastole (rows A&B in the table below), the A row represents the diastolic cells that are not captured by the forcing pulse. If 50% of the heart's cells are in diastole and 40% of those are not captured that is 20% of the total cells. These cells will, however, shortly contract on their own (from previous wavefronts or new ones) providing a positive gain in mechanical action and therefore cardiac output. The B row corresponds to the diastolic cells that are captured. If 60% of the diastolic cells (50% of total) contract due to the forcing field this is 30% of the total heart cells. These cells provide the biggest gain in mechanical action and cardiac output. Next consider the activity of the systolic cells (rows C&D). If 50% of the heart's cells are in systole and 80% of those are not captured (row C), that is 40% of the heart's cells. These cells soon relax and negate a portion of the cardiac output. The systolic cells that are captured (row D) are 10% of the heart's cells (20% of 50%). These cells will hold their contraction and be neutral to cardiac output. The net result (Rows A, B, C, and D) is a gain in contraction which forces cardiac output.

| **Original Status of the Cells** | **Percentage of the Cardiac Cells** | **Status of the Cardiac Cells** | **Percentage of the Original Status** | **Percentage of the Total Cells** | **Mechanical Action** | **Forcing Cardiac Output Effect** |
|---|---|---|---|---|---|---|
| (A) Diastolic | 50% | Diastolic non-captured | 40% of 50% | 20% | will start to contract on own | positive (+) |
| (B) Diastolic | | Diastolic captured | 60% of 50% | 30% | contract | positive (++) |
| (C) Systolic | 50% | Systolic non-captured | 80% of 50% | 40% | will start to relay on own | negative (-) |
| (D) Systolic | | Systolic captured | 20% of 50% | 10% | hold | neutral (0) |
| **TOTAL** | **100%** | | **100%** | **100%** | **more contraction** | **positive (+)** |

The net result over a 200 ms mechanical response is given in the next table. The major contribution is in row (B) from the captured diastolic cells contracting.

| **Row** | **Status of the Cardiac Cells** | **Change in Output** | **Description of Activity** |
|---|---|---|---|
| A | Diastolic non-captured | +5% | Positive. Some cells will begin to contract on their own. |
| B | Diastolic captured | +30% | Positive. Cells contract due to forcing field |
| C | Systolic non-captured | -5% | Negative. Cells begin to relax on own. |
| D | Systolic captured | 0% | Neutral. Cells hold contraction due to forcing field. |
| **Net Gain** | | **+30%** | **A net gain in cardiac output due to forcing fields.** |

The 30% net pumping action should be sufficient to maintain survival and consciousness, because the heart has a 4 - 5 times reserve capacity.

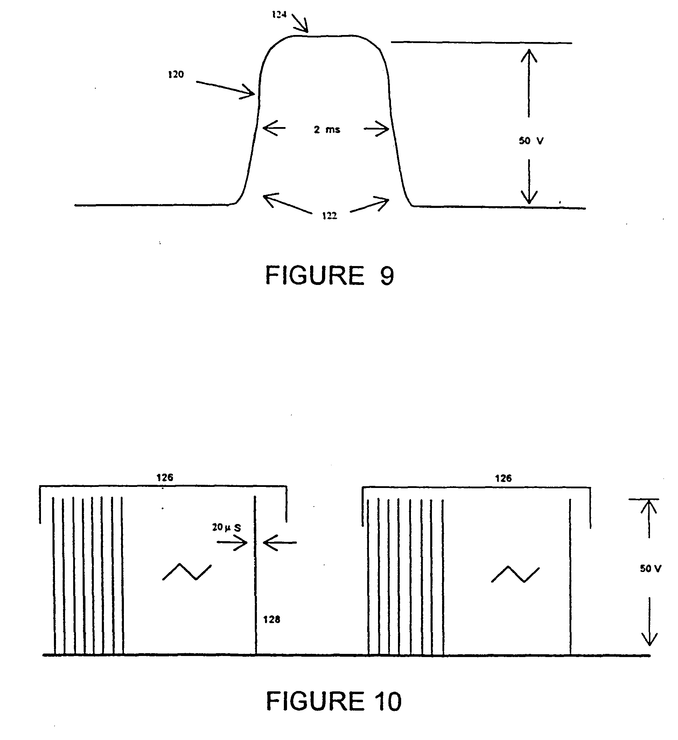

**FIG. 9** depicts examples of waveforms designed to minimize the twitching of the chest muscles which can be very uncomfortable to the patient. A low harmonic pulse waveform 120 which has a very gradual "foot" 122 and a gradual peak 124 has less high frequency components and thus is less likely to stimulate the skeletal muscle.

**FIG. 10** shows a technique of going to the opposite extreme Here, each compound forcing pulse 126 is actually composed of 50 very short spikes 128 each of which is 20 µs in width with a 20 µs spacing. The heart will tend to average out these thin pulses and "see" a 2 ms wide forcing pulse. The skeletal muscle, however, is not efficiently stimulated by these extremely narrow pulses. The skeletal muscle will not average out this signal either This approach could help minimize skeletal muscle twitching and discomfort.

An alternative system would be to charge the capacitor to 300 V for the first pulse to capture many cells therefore putting those cells into diastole after a delay of 100 - 200 ms. At this point the voltage could be lowered to 100 V and pulses delivered every 100 ms. A 3 watt DC-DC converter with a 67% efficiency could provide 100 ms interval forcing pulses assuming a 50 Ω resistance and 1 ms pulse (0.2 J). This rate is too fast for forcing cardiac output due to mechanical limitations, but is very effective for electrical capture. After sufficient capture, the rate of forcing pulses could be slowed down to 100 - 170 beats per minute for optimum cardiac output.

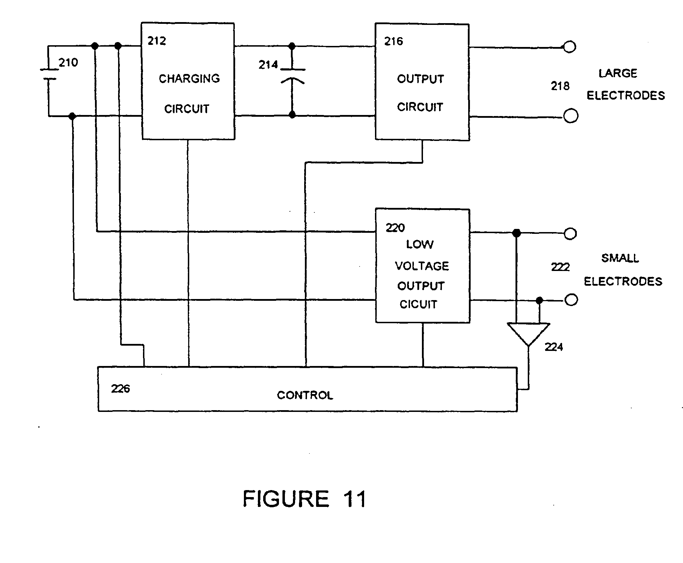

FIG. 11 is a block diagram of one embodiment of the invention. Battery 210 is used to provide power for all circuitry in this embodiment. This includes the charging circuit 212 which provides the means to charge up capacitor 214 in the event that ECOF (electrical cardiac output forcing) is required. The output circuit 216 is then used to deliver the electrical current pulses from the energy stored in capacitor 214 to the large cardiac electrodes 218.

The battery 210 also provides the power to the low voltage output circuit 220 which in turn provides pacing pulses to the small cardiac electrodes **222**. These small cardiac electrodes are also used to sense the cardiac activity which is then run through amplifier 224 and thence delivered to the control unit 226. This control unit is also responsible for controlling the charging circuit 212 and both the higher output voltage circuit 216 and the low voltage output circuit 220.

In a typical operation the signal from the small electrodes 222, after being amplified by amplifier 224 will be monitored by the control unit 226. If that control unit senses only normal rhythm then nothing is done. If, however, the control unit were to sense a ventricular tachycardia then it would initiate antitachycardia pacing through the low voltage output circuit 220 and small electrodes 222. The formulas for calculating the timing of such ATP output pulse trains are well known in the art as listed in the background section. (An example is also discussed later in conjunction with FIG. 7.) The small electrodes, amplifier and control unit are also capable of diagnosing VF. If the antitachycardia pacing does accelerate the heart into a VF then this will be noted by the device. In that case the control unit 226 will immediately initiate the charging of capacitor 214 by charging circuit 212, the control unit 226 will also control the delivery of higher voltage pulses by means of output circuit 216 into the large cardiac electrodes 218. The application of these pulses with a typical ampitude of 30-200 V at a rate of 100-150 pulses per minute (typically) will be sufficient to generate repeated partial contractions of the heart. These partial contractions should be sufficient to maintain consciousness on the part of the patient. The patient can then call 911 or alert a bystander to provide transportation to a hospital. With the use of external defibrillation the patient should be restored to a normal rhythm at that point.

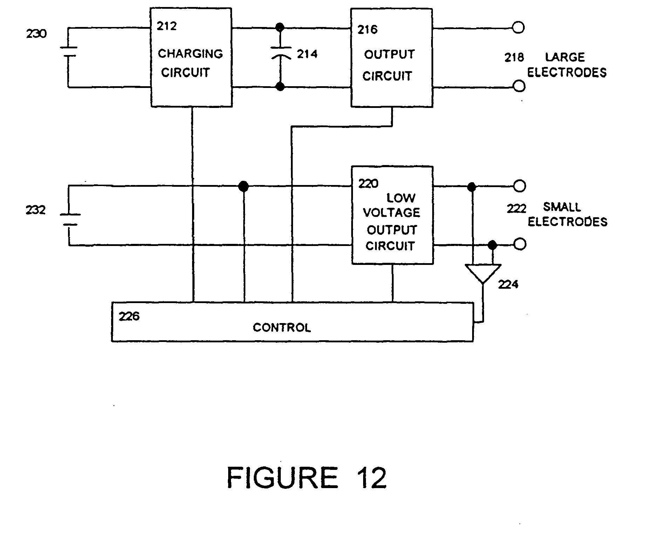

**FIG. 12** shows an embodiment which is identical except for the provision of two different battery energy sources. Battery 230 which provides power for the charging circuit 212 would have to be of a higher current output cell. The typical implantable battery chemistries which have appropriate performance are lithium silver vanadium oxide, titanium carbon monofluoride, or thionyl chloride. The battery 232 which provides the power for the low voltage output circuit 220 and the control unit 226 could be of a lower current output type such as a lithium iodine cell.

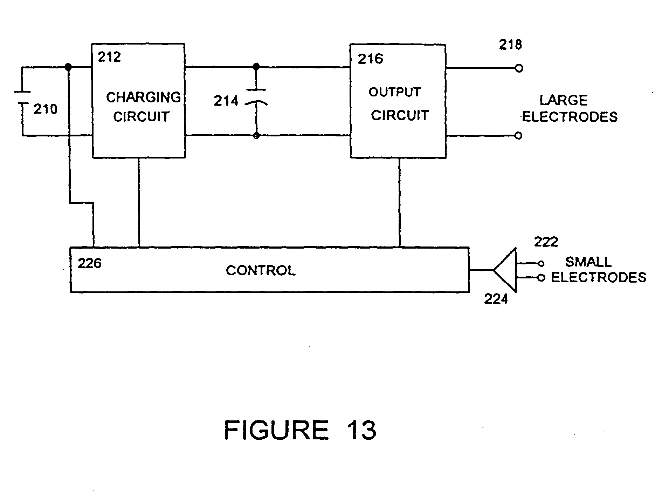

FIG. 13 shows an alternative embodiment in which the antitachycardia pacing is done through the large electrodes 218 rather than through the small electrodes 222. With this approach the output circuit 216 is controlled to deliver voltages on the order of 5-30 volts for the initial antitachycardia pacing. In the event that this pacing was unsuccessful then higher voltage pulses could be used and delivered through the large electrodes 218. The use of large electrodes for antitachycardia pacing is taught in U.S. # 5,330,509 of Kroll entitled "Far Field Antitachycardia Pacing." However, that invention did not anticipate the use of the electrical cardiac output forcing backup. In this embodiment of the instant invention, the small electrodes are still used for sensing the rhythm in order to make the correct diagnosis of VT or VF.

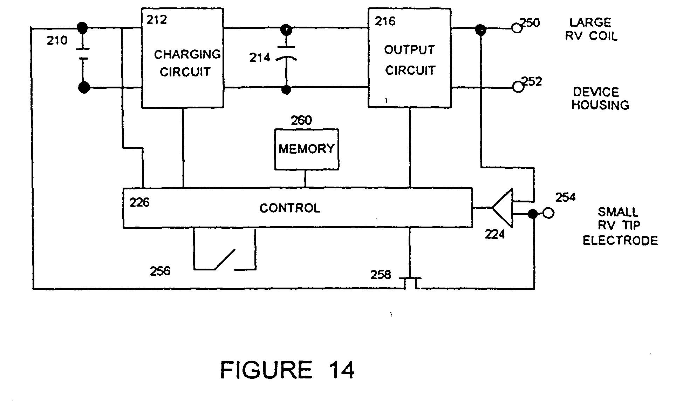

**FIG. 14** shows a preferred embodiment for the invention. The large electrodes are specifically a large right ventricular coil 250 with a longest linear dimension of at least one centimeter and preferably 2-8 centimeters. The other electrode for the ECOF backup is the device housing itself 252. A single small right ventricular tip electrode 254 is used for sensing with the large right ventricular coil 250 being the reference for the amplifier 224.

A magnetic reed switch 256 is monitored by the control unit and all output ceases with the application of a strong magnet over the patient's device. This could be used by the patient to stop the output but more likely by emergency personnel to turn the device off in order to more effectively perform external defibrillation.

Memory 260 is used to store programmable parameters, patient history, and patient stored electrograms.

Finally, the control unit uses transistor switch 258 to gate low voltage pacing pulses from the battery 210 to the small right ventricular tip electrode 254 for antitachycardia pacing. This could also be used for bradycardia pacing (therapy for patients with slow heart rates in the ventricle). It is common practice to use a negative voltage (cathodal) stimulation for such pacing. If desired, the conversion of the positive voltage from battery 210 to a negative pacing pulse is a trivial exercise for anyone skilled in the art.

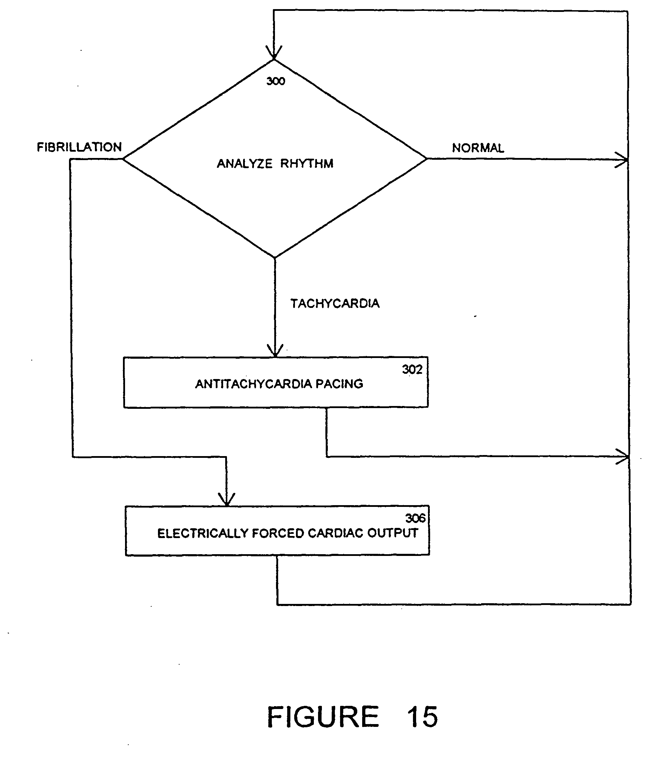

**FIG. 15** depicts the basic method of the invention. In step 300 the device senses and analyzes the rhythm. If a normal rhythm is sensed it simply stays in a waiting mode. If VT is sensed then the method proceeds to step 302 which is to perform antitachycardia pacing. After each attempt of antitachycardia pacing step 300 is used to analyze the rhythm. If the rhythm has returned to normal, then the method returns to monitoring. If VT is still sensed then the device again tries to perform ATP. If VF is sensed then the device proceeds to step 306 to force cardiac output electrically.

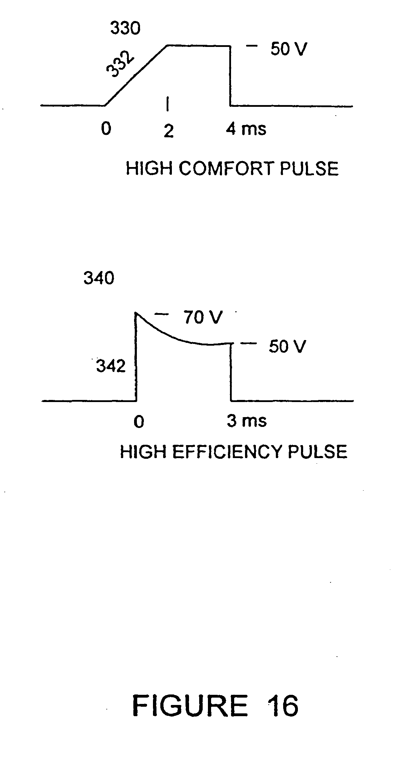

**FIG. 16** depicts some of the pulses that are possible with such a device. Pulse 330 is a high comfort pulse. This pulse uses two milliseconds to climb from zero volts to full voltage. This full voltage is shown as 50 V in this example. The full voltage then is maintained for another 2 ms. This slow ramp 332 in the first 2 ms is less likely to stimulate nerve cells and skeletal muscles thus resulting in significantly less discomfort for the patient. However, this high comfort pulse is relatively inefficient as the output circuitry 216 (FIG. 14) must lose some energy (convert it to heat) by gradual turning on and thus is able to deliver fewer total pulses.

The high efficiency pulse 340 is generated by merely directly connecting the capacitor 214 (FIG. 14) through to the output electrodes 218 (FIG. 14) by means of a direct switch in the circuitry 216 (FIG.14). The voltage then follows the exponential decay of the capacitor which is shown here, as an example, decaying to 50 V over a period of 3 ms. Such a pulse is highly efficient in that essentially no energy is wasted in the output circuitry. However, the high frequency spectral content from the fast rising edge 342 can cause a great deal of patient discomfort.

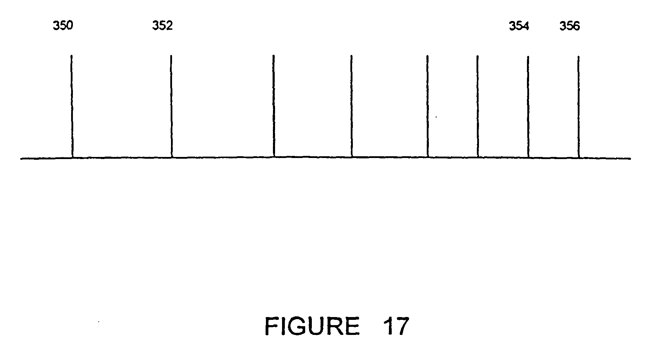

**FIG. 17** shows a typical antitachycardia pacing burst. The burst has 8 pulses in it. This type of burst is known as a "ramp" as the rate of the pulses increases during the burst. Note that the spacing between the first two pulses 350 and 352 is much greater than that between the last two pulses 354 and 356. A typical spacing for pulses between 350 and 352 is 95% of the spacing between cycles of the patient's ventricular tachycardia. However there are many other formulas for calculating these spacings and the size, number, and characteristics of such bursts which are very well covered in the literature and well known to those skilled in the art.

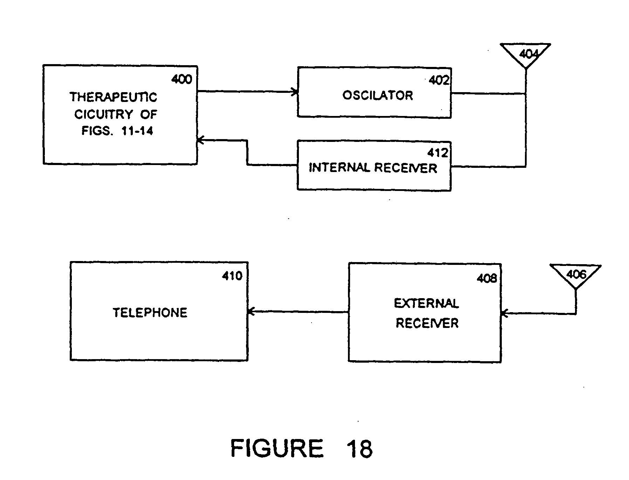

**FIG. 18** shows a complete system for the use of this device. The therapeutic circuitry shown in FIG. 11 through FIG. 14 is shown as system block 400. Added to this is an oscillator 402 which is used to transmit a signal through antenna 404. Antenna 404 could be a coil within the device, one of the defibrillation leads itself, or a separate antenna in the patient's body. That signal is then received by antenna 406 and processed by external receiver 408. That external receiver is then connected to a telephone 410.

In one embodiment the external receiver sits in the patient's place of living and is connected to the patient's home telephone. In another embodiment the external receiver is a small, very portable unit which is connected to a cellular phone. In operation, when the device 400 detects a VF, it then sends a signal through oscillator 402, antenna 404, antenna 406, and external receiver 408. The external receiver 408 generates a voice message which is channeled through telephone 410 to call a physician's office or the appropriate emergency authorities alerting them that the patient will need defibrillation within the next approximately 1 hour. The external receiver 408 could be made relatively sophisticated and could, for example, generate a detailed fax of the patient's condition which is then transmitted to the nearest emergency room. This fax could also include the various electrical signals recorded from inside the patient's heart. The use of fax transmission is taught in U.S. # 5,336,245 of Adams et al. Alternatively, it could call a large battery of numbers such as the local emergency room, rescue squad, and patient's physician. It could also call relatives and neighbors who could be enlisted to provide transportation to the hospital.

The internal receiver 412 is used to convey programming parameters and operational commands from a physician.

There are many choices available for the battery. One choice would be the lithium silver vanadium oxide battery which is abbreviated SVO. This is the battery that is used in all present ICDs. It has the advantage of being able to deliver power at a very high rate. Its disadvantages are that its energy density is only about on the order of 1,000 joules per cubic centimeter (depending upon the construction) and that it is very expensive. The titanium carbon monofluoride battery has the advantage of an energy density on the order of 2,000 joules per cubic centimeter while the disadvantage is that it is only capable of delivery of about 1 watt of power while the SVO cell can deliver at a rate of at least 6 watts depending upon the construction.

The following table gives some examples of device lifetime and its dependence on the various factors of the output voltage, impedance, pulses per minute, and the battery capacity in joules. The voltage is practically limited to 375 V which the maximum rating for a modern photoflash capacitor.

| Voltage | Pulse width (ms) | Impedance | Energy/ Pulse | Pulses Per Min | Watts Ave | Battery Cap joules | Minutes Backup |
|---|---|---|---|---|---|---|---|
| 50 | 3 | 100 | 0.075 | 120 | 0.15 | 5000 | 556 |
| 100 | 3 | 100 | 0.3 | 120 | 0.6 | 5000 | 139 |
| 200 | 3 | 100 | 1.2 | 120 | 2.4 | 10000 | 69 |
| 30 | 3 | 100 | 0.027 | 150 | 0.0675 | 2000 | 494 |
| 70 | 3 | 100 | 0.147 | 150 | 0.3675 | 5000 | 227 |
| 100 | 3 | 100 | 0.3 | 120 | 0.6 | 5000 | 139 |
| 70 | 3 | 50 | 0.294 | 120 | 0.588 | 2000 | 57 |
| 70 | 3 | 50 | 0.294 | 60 | 0.294 | 2000 | 113 |

The calculations ignore converter inefficiencies, which will reduce backup time, yet do use conservative battery ratings, which will increase backup time. In all cases we assume a fixed pulse width of 3 milliseconds. In the first case ECOF pulses of 50 volts are used and the large electrode impedance is 100 ohms using an ECOF pulse rate of 120 pulses per minute and a battery capacity of 5,000 joules. The current would be 500 mA. There are 556 minutes of backup available, or in other words, nearly 10 hours. This is clearly excessive as the patient would find 10 hours of backup very uncomfortable and there are very few places in industrialized society in which the patient could not receive defibrillation within one hour. In the second case, we have a patient requiring a voltage of 100 volts to maintain adequate output. With everything else being the same, this reduces the minutes of backup to 139. It is conceivable that a patient could have a need for a relatively large voltage to maintain minimum cardiac output. An example given here is 200 volts. The current would increase to 2,000 mA. The battery capacity would have to be increased to 10,000 joules in order to maintain a 1 hour backup which is shown here as approximately 69 minutes.

Many patients should have sufficient cardiac output with 30 volt pulses. This would require a current of only 300 mA. If the rate of the ECOF pulses is increased to 150 pulses per minute to maximize output, then the battery capacity could be reduced down to 2,000 joules and the patient would still have 494 minutes of backup. If a patient needed a 70 volt pulse and a rate of 150 pulses per minute to maintain output and the earlier discussed battery capacity of 5,000 joules was used then there would be nearly 4 hours of backup available (actually 227 minutes). If this patient required 100 volt pulses and the pulsing rate was reduced to 120 pulses per minute, then the minutes of backup from the same battery would be reduced to 139, or a little over 2 hours.

In what should be the typical case, the patient will receive a 70 volt pulse at a rate of 120 pulses per minute with a small battery with only 2,000 joule capacity. This should give 57 minutes of backup. If the same patient were to have a rescue delayed then the pulse rate could be reduced to 60 per minute which would extend the minutes of backup to 113 or nearly 2 hours. As can be seen, there is a great deal of flexibility in the programming of the output voltage and the choice of battery capacity for different patients. A patient in an urban environment could get by with a very small battery while one that is in a rural environment might require a larger battery and an automatic telephoning system.

A 2,000 joule titanium carbon monofluoride battery should be on the order of 1 cubic centimeter in volume. If this battery were of the SVO type then its volume would be at least 2 cubic centimeters.

The capacitor could also be made very small. Assuming a 60 microfarad capacitance, even with the 200 volt maximum output shown in the table, the total stored energy would only be 1.2 joules. Modern electrolytic capacitors have an energy density of about 1 joules per cubic centimeter and thus this capacitor would have a volume of approximately 1 cm³. Thus the total volume of the components for the energy (namely the battery and capacitor) would have volumes on the order of 2 or 3 cm³ compared to volumes of 20-30 cm³ or more in the present ICDs.

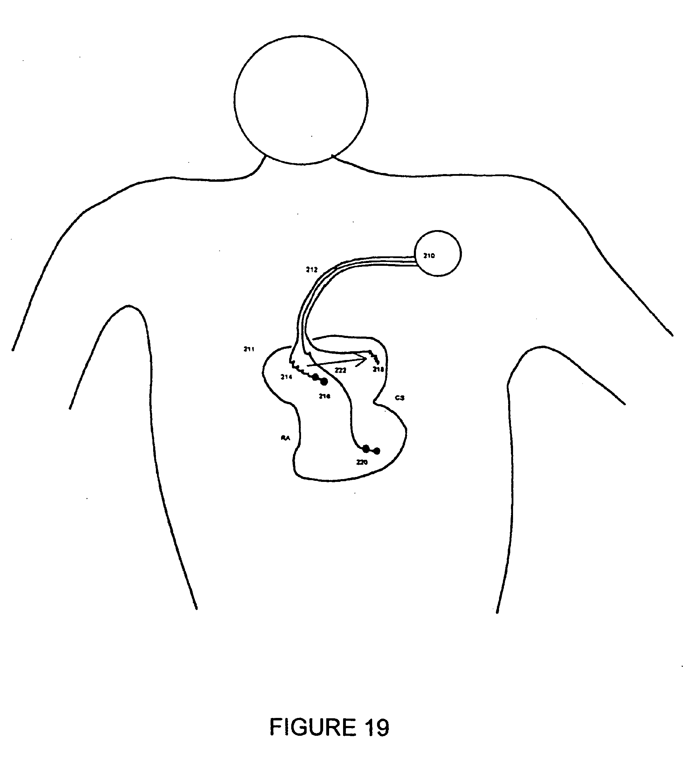

**FIG. 19** shows the basic atrial defibrillator described with reference to Figures 1 to 10 implanted in the human body. Here the electronic system is enclosed in housing 210 with a lead set 212 connecting to the heart 211. Note that the housing is preferably implanted in the left ventricle region of the patient.

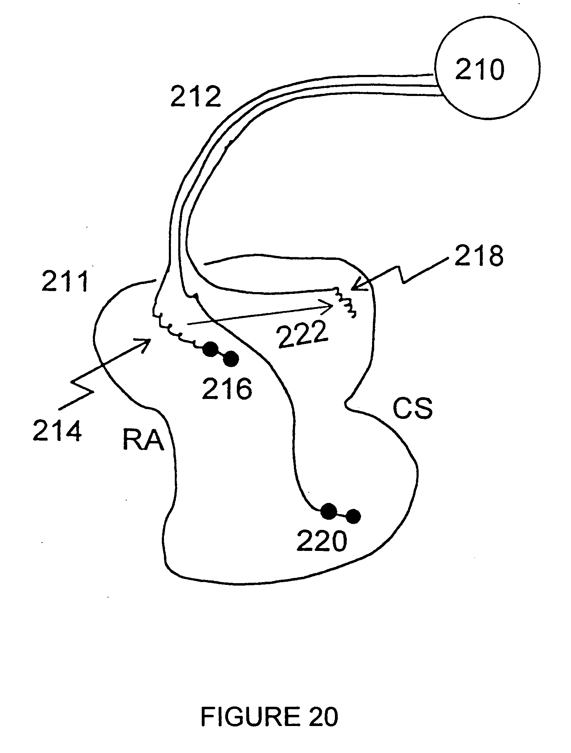

**FIG. 20** shows a detailed view of the leads used for the implantable atrial defibrillator. An atrial lead coil 214 is used for shock delivery and sensing pair 216 is used for sensing the rhythm in the right atrium. The lead coil 214 is typically placed in the right atrial appendage for optimum shock current delivery. Another coil 218 is located in the coronary sinus. Thus these coils are as far apart as they can be practically spaced inside atria. The atrial defibrillation shock is delivered between the coils 214 and 218 producing the electrical current path 222.

A bipolar sensing pair 220 is located in the right ventricle. This sensing pair is used for many purposes, but primarily for synchronization to minimize the risk of delivering the atrial defibrillation shock in the middle of the ventricular T-wave.

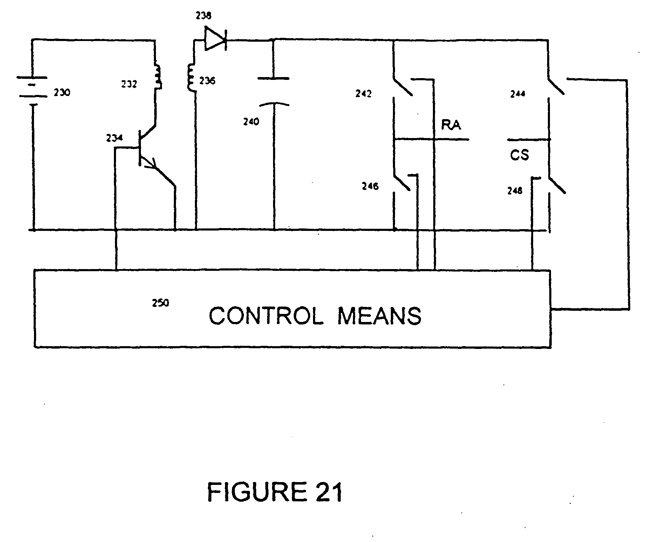

**FIG. 21** gives a schematic of the conventional atrial implantable defibrillator. Battery 230 is used to deliver current through flyback transformer primary 232 on an interrupted basis which is caused by transistor switch 234 being controlled by the control means 250. The electrical output from a transformer is delivered by secondary winding 236 through diode 238 and stored in high energy capacitor 240 The energy in the high voltage capacitor 240 is delivered to the heart electrodes of the right atrium and coronary sinus through an "H bridge" circuit. For delivery of the positive portion of the biphasic wave switches 242 and 248 arc turned on thus passing current from the RA (right atrial) lead to the CS (coronary sinus) lead After a brief period of time those switches are opened then, switches 244 and 246 are turned on so that the current is passed into a reverse direction creating a biphasic pulse.

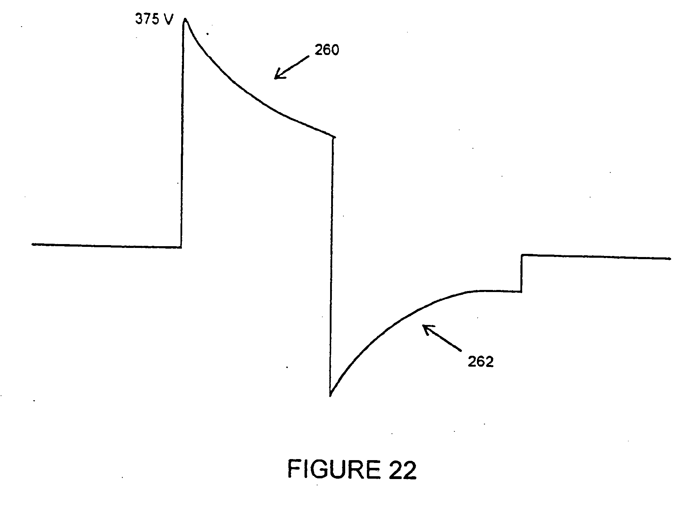

**FIG. 22** shows the biphasic waveform generated by the circuitry in **FIG. 20** The first (or positive) phase 260 begins at a 375 volt maximum. After this phase is completed a negative phase 262 begins.

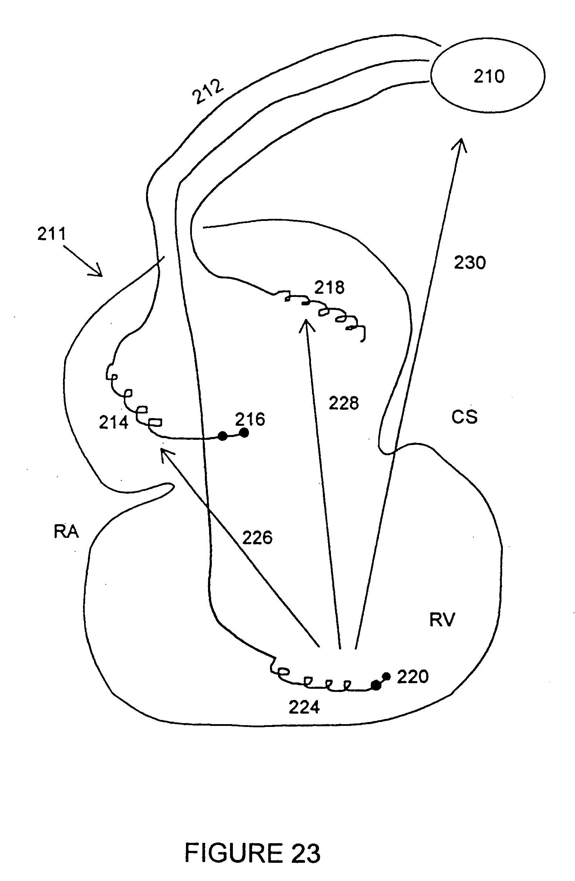

**FIG. 23** shows the lead system in one embodiment for the instant invention. Two electrodes are added to the conventional lead system for an implantable atrial defibrillator. These are a right ventricular coil 224 and the device housing 210 which is now connected electrically to function as another electrode. Atrial defibrillation is performed as before for the implantable atrial defibrillator of the conventional design. However, in the event that the delivery of the atrial defibrillation shock led to ventricular defibrillation then the electrical cardiac output forcing operation is begun using the coil 224 in the right ventricle. In the simplest configuration the right atrial coil 214, the coronary sinus coil 218, and the Can housing 210 are all connected in parallel The right ventncle thus passes current simultancously along current paths 226, 228, and 230.

Alternatively the upper electrodes could be cycled to provide a more varied electric field across the heart to possibly engage more of the heart cells in contributing to the partial ECOF contraction. For example, the right atrial lead 214 could be operated separately. In this embodiment the right atrial lead would receive every second ECOF pulse. The intervening ECOF pulses would be delivered to the parallel combination of the coronary sinus 228 and the Can as an electrode 210.

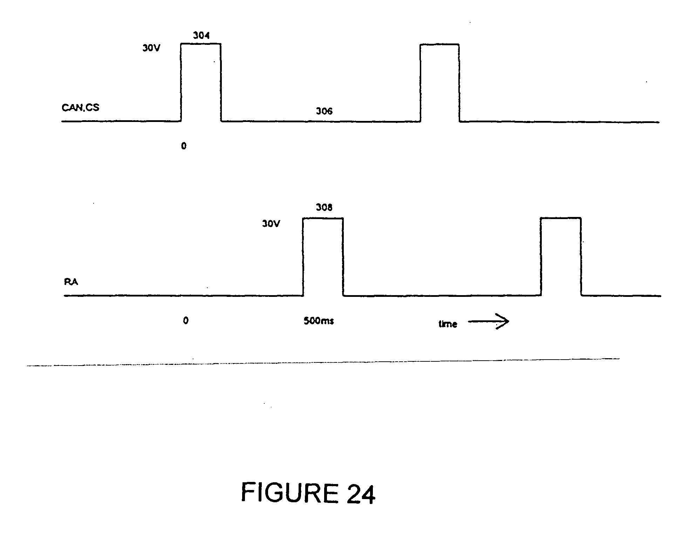

FIG. 24 shows the waveforms when the Can and coronary sinus leads are connected together, but the RA is separate. In this case, those two electrodes receive a 30 V pulse 304 every 1 second. In the middle of the intervening space 306 between these pulses, the right atrium receives a 30 V pulse 308.

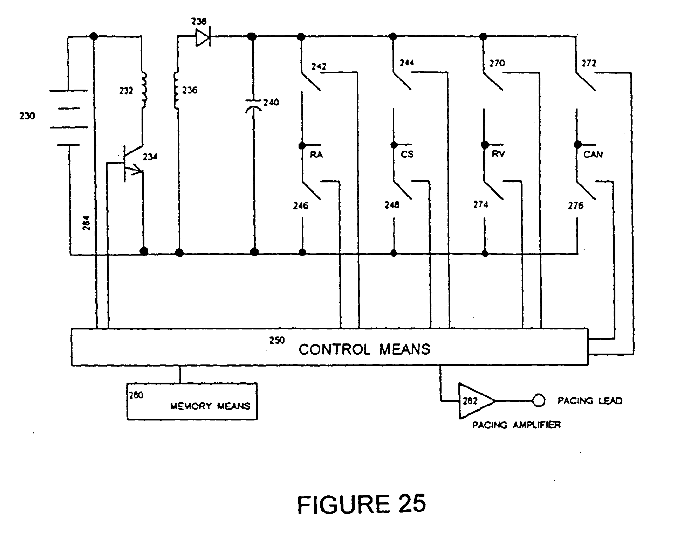

**FIG. 25** depicts the basic schematic of the instant invention. This is very similar to the implantable atrial defibrillator except for two significant departures. First of all there are distinct controls for the right ventricular coil and the device "Can." The right ventricular coil is controlled by switches 270 and 274 while the Can electrode is controlled by switches 272 and 276. The separate switches allow for timing and pulse width variations. For example, the RA and CS switches could conduct current prior to the RV and CAN assisting in atrial output prior to ventricular ECOF. The RA and CS switches could also have a shorter pulse width so the majority of the current is transferred between the RV and CAN.

Another significant difference is that the control means allows for the use of moderate continuously generated voltages of a capacitor 240 by the continuous operation of transistor switch 234. This keeps the inverter running so a moderate voltage is maintained on capacitor 240 for a continuous delivery of the ECOF pulses. Alternatively the transistor 234 could be run at a lower duty cycle so that the voltage across capacitor 240 is minimized.

The control means 250 is connected to memory means 280 to allow the storage of programming parameters and electrograms. Also pacing amplifier 282 allows the delivery of bradycardia pacing pulses. Battery connection 284 allows the control means to monitor the battery voltage at all times thus determining its status.

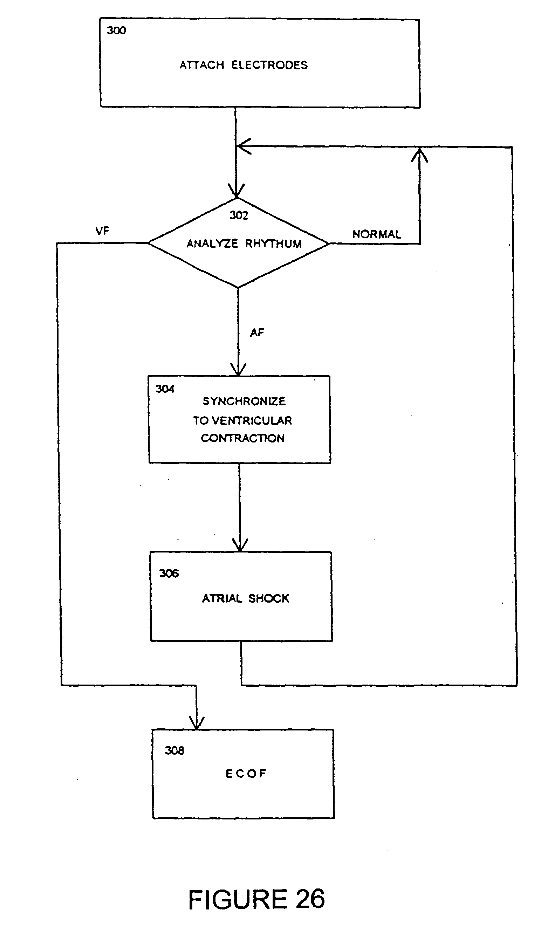

**FIG. 26** depicts the primary method of the invention. First electrodes are attached 300 and then the rhythm is analyzed 302. If ventricular fibrillation is detected then electrical cardiac output forcing would be performed 308. If atrial defibrillation was detected then the ventricular contraction would be synchronized to 304 and an atrial shock delivered 306. In ECOF step 308, the voltage and therapy delivery can be varied. The voltage is varied based on the level of cardiac output. The pulse shape based on battery level.

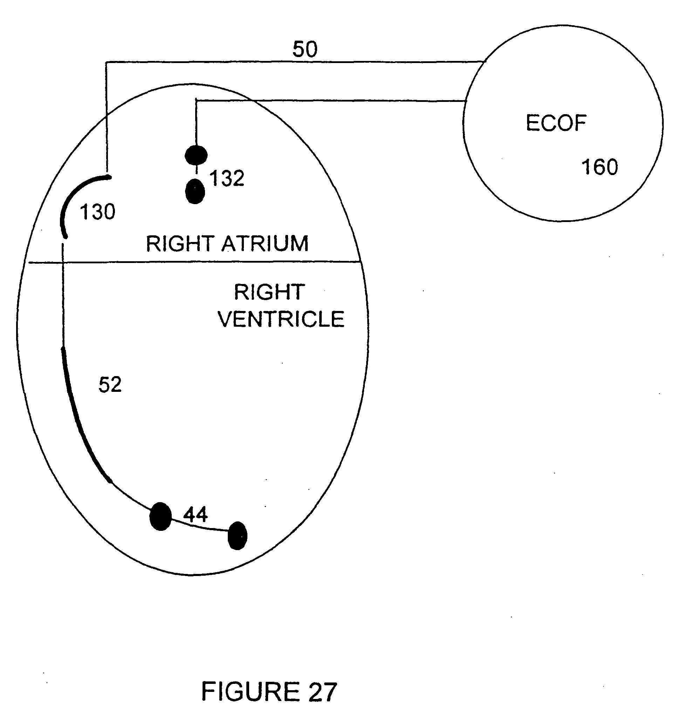

**FIG. 27** shows another system in which the invention is embodied. This is similar to the system described with reference to Figures 1 to 10, but in this case a right atrial coil electrode is provided. If the atria are also in tachyarrhythmia (assuming the ventricles are), the right atrial coil delivers a current pulse at a fixed interval prior to the main ventricular pulse delivered by the right ventricular coil 52. By synchronising the ventricular forcers thusly, the output of the atria is employed which can increase total cardiac output by as much as 20%.

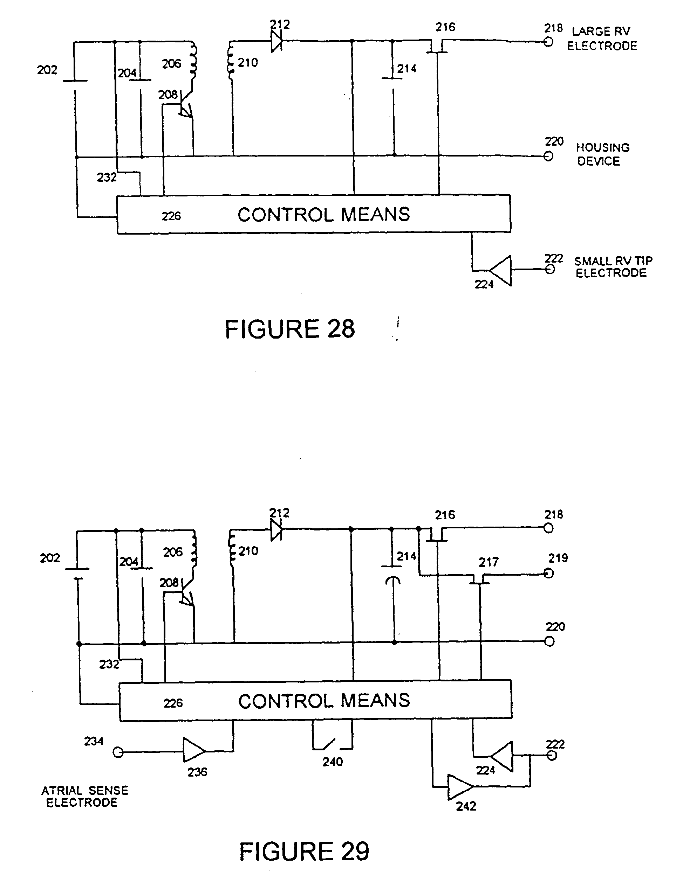

**FIG. 28** shows a basic schematic of the system. A high capacity implantable grade battery 202 is used to power the overall system. This battery could be of any high capacity lower impedance type of implantable cell. This would include lithium silver vanadium oxide, thionyl chloride, or titanium carbon monofluoride for example. Various cells have different advantages. The lithium silver vanadium oxide cells have extremely powerful outputs but rather limited energy densities. The titanium carbon monofluoride cells have a lower power output but have twice the energy density of the lithium silver vanadium oxide cell. The cell (or battery of cells) need be capable of providing at least 100 mW to deliver pulses of sufficient voltage and rate to temporarily maintain cardiac output.

The output voltage from the cell is smoothed by small capacitor 204. The power from the cell 202 is then used to deliver a current through flyback transformer primary winding 206. That current is generated on an interrupted basis through winding 206 by the operation of switching transistor 208 which is controlled by control means 226. The output energy from the flyback transformer is directed through secondary winding 210 and passes through diode 212 and is stored in capacitor 214.

A typical specification for capacitor 214 is 60 µF and 70 V. The time constant with a load of 100 Ω and the 60 µF (microfarads) capacitor is 6 ms (milliseconds) which is truncated to produce a reasonably level 3 ms wide forcing pulse. Capacitors as small as 10 µF would also work although they would deliver a higher voltage and more narrow pulse while capacitors with values of 200 µF would also be usable. In the case of the 60 µF capacitor and a maximum output voltage of 70 V the energy can be calculated by the formula E = 1/2 C V² giving a total energy of 0.15 J. This is an extremely small capacitor compared to the typical 30-40 J capacitor which is the total capacitive storage in an ICD. Even if the ECOF was designed to operate to voltages of 200 volts which are immensely practical then the total energy storage of the capacitor would only be 1.2 J. With the rule of thumb that high density aluminum electrolytic capacitors are capable of storing about 1 J per cm³ the capacitor would have a total volume of only about 1 cm³. This is in sharp contrast to the very large capacitors of present ICDs which are on the order 20 cm³. This is one of the reasons why the ECOF can be made much smaller than the an ICD.

Pulses are delivered to the heart from the energy stored in capacitor 214 by the operation of output switch 216 which is controlled by control means 226. Those pulses are then delivered to an electrode in or near the heart which is typically a large right ventricular coil which is connected to terminal 218. The other pole for the heart current would typically be the device housing itself connected to terminal 220. The fibrillation of the heart is detected by the use of a small right ventricular tip electrode connected to terminal 222 whose signal is then amplified by amplifier 224 and then fed into the control means. This amplifier is capable of recognizing ventricular fibrillation or a high rate ventricular tachycardia and thus initiating, through the control means, the electrical cardiac output forcing. The battery voltage is monitored through connection 232 by the control means. This allows the control means to be aware of the state of the battery charge. The control means can thus signal for a change in the device or switch to a more efficient waveform with a decreased voltage on the battery.

**FIG. 29** shows a more complicated embodiment of the system.

Connection 234 goes to a sense electrode in the atrium. This signal is then run through amplifier 236 and then to the control means. The sensing of the atrial signal is useful for a number of reasons. One reason is that by monitoring the electrogram from both the ventricle and the atrium, the control means can make a more intelligent decision about the presence or absence of a ventricular fibrillation or ventricular tachycardia. Secondly, the atria are responsible for about 20% of the cardiac output. By sensing the atrial contraction, and synchronizing to that, the ECOF should be able to increase its output efficiency. The lead in the right atrium could also provide ECOF pulses prior to ventricular ECOF pulses, if the atrial are also fibrillating. This would increase cardiac output even more. The atrial coil is connected to terminal 219 and connected to switch 217 and controlled by control means 226.

Magnetic reed switch 240 allows a patient or physician to terminate the (possibly uncomfortable), ECOF pulsing by placing a strong magnet over the chest. That magnetic field is detected by the magnetic switch 240 which then inhibits the electrical output Alternatively the sensor could be a Hall effect or MAGFET magnetic sensor.

Output amplifier 242 is used to generate pacing pulses on the order of the battery voltages These pulses have an amplitude of typically less than 10 V. They are delivered to the heart through the same electrode which is connected to node 222. This feature is necessary to those high-risk patients which are also suffering from a bradycardia. Since the ECOF device is closer in size to a conventional bradycardia pacemaker, the physician can easily implant it in pacing patients and gain the lifesaving benefits of ECOF in the event of a cardiac arrest.

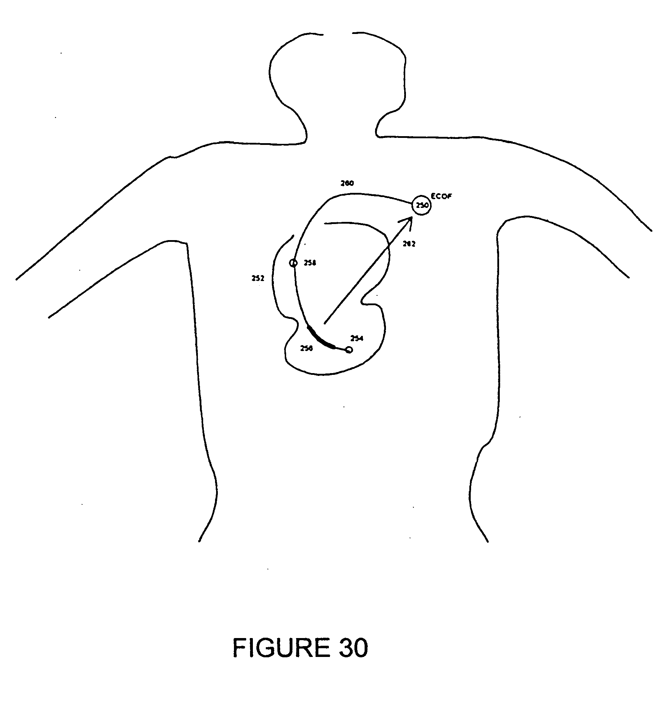

**FIG.30** shows the ECOF 250 implanted in a left pectoral region of the patient. Lead 260 is then run through a vein and down into the right atrium and right ventricle of the heart. The right ventricular tip electrode 254 is used to sense the electrical activity to diagnose fibrillation and is connected to terminal 222 (in **FIG. 28**). A larger coil electrode 256 is connected to terminal 218 (in **FIG. 28)** and is used to deliver the moderate voltage pulse which will drive current from this coil 256 along a path 262 through the majority of the ventricle and towards the ECOF device 250.

Finally, an atrial sensing lead 258 lies in the atrium to sense the atrial electrogram to aid in diagnosis or synchronizing of the ECOF output.

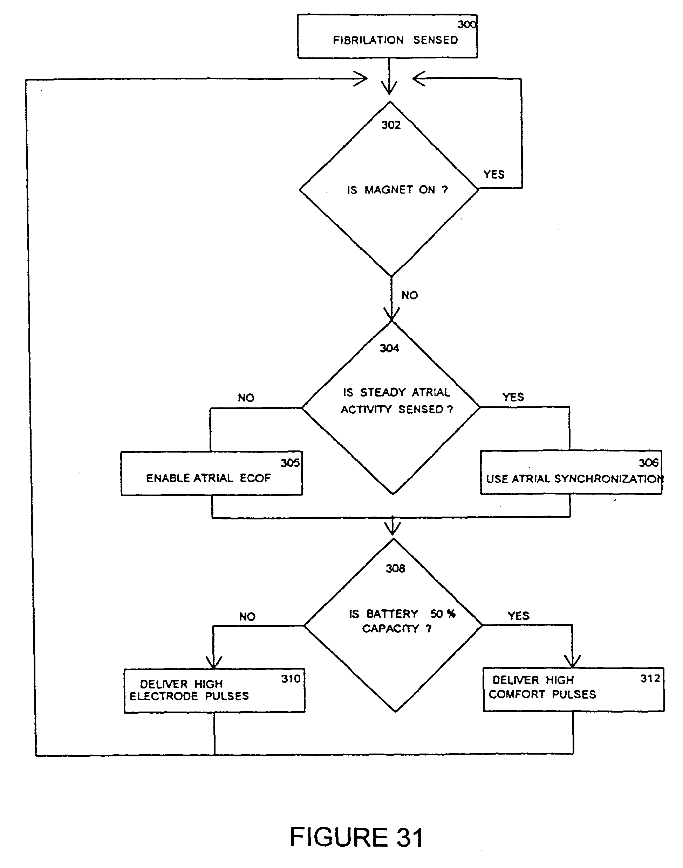

**FIG. 31** depicts the operational sequence for the ECOF device optimized for high-risk cardiac patients. The first step in the method is the sensing of fibrillation 300. After fibrillation is sensed, then the decision 302 is made as to whether or not the magnet is present. If it is, then the device simply waits and performs no additional therapy until the magnet is removed. In the absence of the magnet then the procedure proceeds down and an attempt is made to sense atrial activity on a steady basis 304. If this is successful, then the decision is made to use atrial synchronization as shown in box 306. Otherwise atrial ECOF therapy is enabled 305. Step 308 tests the battery capacity. If the battery is still in the early stages of its life, then the ECOF device delivers high-comfort pulses 312 to the ventricle. These pulses are shaped to minimize the high-frequency edges which are typically found in electrical stimulation therapy. Unfortunately these low-spectral content high-comfort pulses are relatively inefficient as much energy must be lost in the output switches such as switch 216 (in **FIG. 28)** in order to shape these output pulses. After the battery has lost half its capacity the unit will automatically shift over towards the delivery of high-efficiency pulses which is step 310.

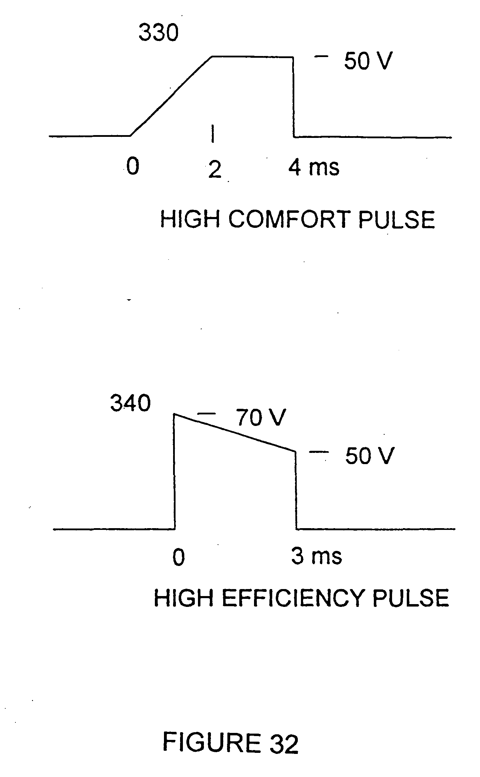

FIG. 32 depicts two very practical sample pulses which the ECOF device could use. Waveform 330 is a high comfort pulse. The waveform begins at 0 volts and gradually climbs to 50 V over a 2 ms period of time. This represents a very gradual voltage change of only 25 V per ms. The pulse then maintains the 50 V for a period of an additional 2ms. This slow rise time on the leading edge makes the pulse much less irritating to the patient and much less likely to stimulate skeletal muscles and nerves. This is generated by slowly turning on output transistor 216 in **FIG.28.** Alternatively, the waveforms in **FIG. 9** or **FIG. 10** could be generated although the control is more complex.

The high efficiency pulse 340 is designed to capture all of the energy from the capacitor without any waste. It thus rises nearly instantaneously to 70 V and tapers to 50 V during the 3 ms width of the pulse then finally returns to 0 V. This is a very efficient pulse in that there are no losses from the switches being used for shaping. It is also a relatively uncomfortable pulse in that the high spectral content of the sharp leading edge tends to stimulate nerves and skeletal muscles in the patient. High comfort stimulation wave forms are taught by Mehra in U.S. 5,0818,522 for an external pacing apparatus.

## Claims

1. Apparatus for temporarily electrically forcing cardiac output, the apparatus comprising:
a battery;
arrhythmia detection circuitry connected to said battery;
a voltage inverter connected to the battery to generate voltage pulses; and
output circuitry, controlled by the arrhythmia detection circuitry, connecting the voltage inverter to a patient's heart to deliver the inverter voltage pulses repetitively in order to directly force a partial contraction in the patient's heart to maintain a minimum level of cardiac output in the event of an arrhythmia;
in which the voltage inverter is arranged to continuously deliver pulses between 30 and 200 volts, at between 60 and 200 pulses per minute, in which the voltage inverter is arranged to deliver a number of pulses between 1,000 and 1,000,000.

2. The apparatus of claim 1 in which the delivery system is external.

3. Apparatus of claim 2 in which the output circuitry turns on gradually and thus provides at least one rounded edge.

4. Apparatus as in claim 2 or claim 3 in which the output circuitry turns on gradually and thus provides a gently sloped leading edge profile.

5. Apparatus as in claim 2, 3 or 4 further comprising means to detect the atrial contraction and means to synchronize its output to the atrial contraction.

6. Apparatus as in any of claims 2-5 in which the battery means includes sufficient storage capacity to provide the forcing pulses for at least one hour.

7. Apparatus as in any of claims 2-6 in which the inverter output voltage is adjusted down to maintain cardiac output while minimizing patient pain and battery drain.
